# EUROPEAN PATENT SPECIFICATION

(11) **EP 2 180 052 B1**
(45) Date of publication and mention of the grant of the patent: **23.01.2013**
(21) Application number: 08791407.3
(22) Date of filing: 22.07.2008
(51) Int. Cl.: C12N 9/10, C12N 9/90, C12P 13/08

(54) **METHOD FOR PRODUCTION OF L-LYSINE**
VERFAHREN ZUR HERSTELLUNG VON L-LYSIN
PROCÉDÉ DE FABRICATION DE LA L-LYSINE

(30) Priority: 23.07.2007 JP 2007190795
(43) Date of publication of application: 28.04.2010
(73) Proprietor: Ajinomoto Co., Inc., Tokyo 104-8315 (JP)
(72) Inventor: DOI, Hidetaka, Kawasaki-shi Kanagawa 210-8681 (JP); UEDA, Takuji, Kawasaki-shi Kanagawa 210-8681 (JP)
(74) Representative: HOFFMANN EITLE
(86) International application number: PCT/JP2008/063126
(87) International publication number: WO 2009/014117

(56) References cited:
- JP-A- 11 192 088
- CREMER J ET AL: "REGULATION OF ENZYMES OF LYSINE BIOSYNTHESIS IN CORYNEBACTERIUM GLUTAMICUM", JOURNAL OF GENERAL MICROBIOLOGY, SOCIETY FOR MICROBIOLOGY, READING, GB, vol. 134, no. 12, 1 January 1988 (1988-01-01), pages 3221-3229, XP008047559, ISSN: 0022-1287
- SCHRUMPF B ET AL: "A FUNCTIONALLY SPLIT PATHWAY FOR LYSINE SYNTHESIS IN CORYNEBACTERIUM GLUTAMICUM", JOURNAL OF BACTERIOLOGY, AMERICAN SOCIETY FOR MICROBIOLOGY, WASHINGTON, DC; US, vol. 173, no. 14, 1 July 1991 (1991-07-01) , pages 4510-4516, XP000884180, ISSN: 0021-9193
- KIM O.M. ET AL.: 'Cloning and Sequencing of the ddh Gene involved in the Novel Pathway of Lysine Biosynthesis from Brevibacterium lactofermentum' J. MICROBIOL. BIOTECHNOL. vol. 5, no. 5, 1995, pages 250 - 256, XP007913418
- YEH P. ET AL.: 'General organization of the genes specifically onvolved in the diaminopimelate-lysine biosynthetic pathway of Corynebacterium glutamicum' MOL. GEN. GENET. vol. 212, 1988, pages 105 - 111, XP008099352
- ISHINO S. ET AL.: 'Cloning and Sequencing of the meso-Diaminopimelate-D-dehydrogenase (ddh) Gene of Corynebacterium glutamicum' AGRIC. BIOL. CHEM. vol. 52, no. 11, 1988, pages 2903 - 2909, XP008099523

## Description

### Technical Field

The present invention relates to a method for producing L-lysine utilizing *Escherichia coli.* L-Lysine is an essential amino acid, and it is utilized as a component of drugs and various nutritional mixtures such as feed additives for animals.

### Background Art

L-Amino acids such as L-lysine are industrially produced by fermentation using amino acid-producing bacteria such as coryneform bacteria and *Escherichia* bacteria having an ability to produce such L-amino acids. As such amino acid-producing bacteria, strains isolated from the nature, artificial mutant strains of such strains, recombinant strains in which L-amino acid biosynthesis enzyme activity is enhanced by gene recombination, and so forth are used in order to obtain improved productivity. Examples of methods for producing L-lysine include the methods described in Patent documents 1 to 4.

As methods for improving an ability to produce amino acid such as L-lysine, besides the method of increasing expression of an enzyme of a biosynthetic pathway characteristic to a target amino acid, there have been developed a method of modifying the respiratory chain pathway to improve energy efficiency (Patent document 5), and a method of amplifying a nicotinamide nucleotide transhydrogenase gene to increase nicotinamide adenine dinucleotide phosphate-producing ability (Patent document 6).

Moreover, as means based on modification of a pathway common to biosyntheses of various amino acids, there are known L-amino acid-producing bacteria in which anaplerotic pathway is modified, such as the L-lysine-producing coryneform bacterium in which pyruvate carboxylase activity is increased (Patent document 7), the pyruvate kinase-deficient L-lysine-producing *Escherichia* bacterium (Patent document 8), and L-lysine-producing coryneform bacterium which is deficient in maleate quinone oxidoreductase (Patent document 9).

As a precursor of L-lysine, there is meso-α,ε-diaminopimelic acid (henceforth also referred to as "meso-DAP"). Meso-DAP is a precursor of L-lysine, and at the same time, it is a substance indispensable for growth of bacteria as a constituent component of cell walls. As for the meso-DAP synthesis in *Escherichia* bacteria, it is known that meso-DAP is synthesized from a precursor thereof, 2,3,4,5-tetrahydropyridine-2,6-dicarboxylic acid (henceforth also referred to as "THDP"), by the functions of four enzymes, 2,3,4,5-tetrahydropyridine-2,6-dicarboxylate N-succinyltransferase (henceforth also referred to as "DapD", Non-patent document 1), succinyldiaminopimelate transaminase (henceforth also referred to as "DapC", Non-patent document 2), succinyldiaminopimelate desuccinylase (henceforth also referred to as "DapE", Non-patent document 3), and diaminopimelate epimerase (henceforth also referred to as "DapF", Non-patent document 4), which belong to the meso-DAP synthesis pathway. It has been clarified that coryneform bacteria have another meso-DAP synthesis pathway which utilizes THDP as a precursor, and synthesize meso-DAP from THDP by one step reaction using meso-DAP dehydrogenase (henceforth also referred to as "diaminopimelate dehydrogenase" or "DDH"), and it is known that expression of DDH is useful for the production of meso-DAP (Patent document 10). Moreover, it was disclosed that, in a process of investigating the rate limiting step of L-lysine biosynthesis in *Escherichia* bacteria, a gene coding for DDH of coryneform bacterium was introduced into an L-lysine-producing *Escherichia* bacterium, instead of enhancing an activity of an enzyme belonging to the meso-DAP synthesis pathway, to perform L-lysine production (Patent document 13). However, it has not been expected that, when DDH is expressed in an *Escherichia* bacterium, decrease of an activity of an enzyme belonging to the meso-DAP synthesis pathway is effective for L-lysine production.
Patent document 1: Japanese Patent Laid-open (KOKAI) No. 10-165180
Patent document 2: Japanese Patent Laid-open No. 11-192088
Patent document 3: Japanese Patent Laid-open No. 2000-253879
Patent document 4: Japanese Patent Laid-open No. 2001-057896
Patent document 5: Japanese Patent Laid-open No. 2002-17363
Patent document 6: Japanese Patent No. 2817400
Patent document 7: Japanese Patent Laid-open based on PCT application in foreign language (KOHYO) No. 2002-508921
Patent document 8: International Patent Publication WO03/008600
Patent document 9: U.S. Patent Published Application No. 2003/0044943
Patent document 10: Japanese Patent Laid-open No. 61-289887
Patent document 11: International Patent Publication WO2006/093322
Patent document 12: U.S. Patent Published Application No. 2006/0160191
Patent document 13: U.S. Patent No. 6,040,160
Non-patent document 1: Richaud, C. et al., J. Biol. Chem., 259 (23):14824-14828, 1984
Non-patent document 2: Heimberg, H. et al., Gene, 90 (1) : 69-78, 1990
Non-patent document 3: Bouvier, J. et al., J. Bacteriol., 174 (16):5265-71, 1992
Non-patent document 4: Wiseman, J.S. et al., J. Biol. Chem., 259 (14):8907-14, 1984

### Disclosure of the Invention

### Object to be Achieved by the Invention

An object of the present invention is to provide an *Escherichia coli* having improved L-lysine-producing ability and a method for producing L-lysine utilizing such an *Escherichia coli.*

### Means for Achieving the Object

The inventors of the present invention conducted various researches in order to achieve the aforementioned object, and as a result, found that L-lysine-producing ability of *Escherichia coli* could be improved by modifying *Escherichia coli* to decrase activity of an enzyme belonging to the meso-DAP synthetic pathway, and introducing a gene coding for diaminopimelate dehydrogenase. The present invention was accomplished on the basis of this finding.

Thus, the present invention is as follows.
(1) An *Escherichia coli* having an L-lysine-producing ability, which has been modified to decrease activity or activities of one or more kinds of enzymes of the meso-α,ε-diaminopimelic acid synthesis pathway, and into which a gene coding for diaminopimelate dehydrogenase has been introduced.
(2) The aforementioned *Escherichia coli*, wherein the enzymes of the meso-α,ε-diaminopimelic acid synthesis pathway are selected from 2, 3, 4, 5-tetrahydropyridine-2, 6-dicarboxylate N-succinyltransferase, succinyldiaminopimelate transaminase, succinyldiaminopimelate desuccinylase, and diaminopimelate epimerase.
(3) The aforementioned *Escherichia coli*, wherein the 2,3,4,5-tetrahydropyridine-2,6-dicarboxylate N-succinyltransferase, succinyldiaminopimelate transaminase, succinyldiaminopimelate desuccinylase, and diaminopimelate epimerase are encoded by the *dapD* gene, *dapC* gene, *dapE* gene, and *dapF* gene, respectively.
(4) The aforementioned *Escherichia coli,* wherein the activities of the enzymes of the meso-α,ε-diaminopimelic acid synthesis pathway are decreased by decreasing expression of the genes or by disrupting the genes.
(5) The aforementioned *Escherichia coli,* which has been modified to decrease at least the activity of 2,3,4,5-tetrahydropyridine-2,6-dicarboxylate N-succinyltransferase.
(6) The aforementioned *Escherichia coli,* wherein the gene coding for diaminopimelate dehydrogenase is the *ddh* gene of a coryneform bacterium.
(7) The aforementioned *Escherichia coli,* wherein the 2,3,4,5-tetrahydropyridine-2,6-dicarboxylate N-succinyltransferase is a protein defined in the following (A) or (B) :
   (A) a protein having the amino acid sequence of SEQ ID NO: 2,
   (B) a protein having the amino acid sequence of SEQ ID NO: 2 including substitutions, deletions, insertions, or additions of one or several amino acid residues, and having the 2,3,4,5-tetrahydropyridine-2,6-dicarboxylate N-succinyltransferase activity.
(8) The aforementioned *Escherichia coli,* wherein the succinyldiaminopimelate transaminase is a protein defined in the following (C) or (D):
   (C) a protein having the amino acid sequence of SEQ ID NO: 4,
   (D) a protein having the amino acid sequence of SEQ ID NO: 4 including substitutions, deletions, insertions, or additions of one or several amino acid residues, and having the succinyldiaminopimelate transaminase activity.
(9) The aforementioned *Escherichia coli,* wherein the succinyldiaminopimelate desuccinylase is a protein defined in the following (E) or (F):
   (E) a protein having the amino acid sequence of SEQ ID NO: 6,
   (F) a protein having the amino acid sequence of SEQ ID NO: 6 including substitutions, deletions, insertions, or additions of one or several amino acid residues, and having the succinyldiaminopimelate desuccinylase activity.
(10) The aforementioned *Escherichia coli,* wherein the diaminopimelate epimerase is a protein defined in the following (G) or (H):
   (G) a protein having the amino acid sequence of SEQ ID NO: 8,
   (H) a protein having the amino acid sequence of SEQ ID NO: 8 including substitutions, deletions, insertions, or additions of one or several amino acid residues, and having the diaminopimelate epimerase activity.
(11) The aforementioned *Escherichia coli,* wherein the *dapD* gene is a DNA defined in the following (a) or (b):
   (a) a DNA comprising the nucleotide sequence of SEQ ID NO: 1, or
   (b) a DNA which is able to hybridize with the nucleotide sequence of SEQ ID NO: 1 or a probe which can be prepared from the nucleotide sequence under stringent conditions, and coding for a protein having the 2,3,4,5-tetrahydropyridine-2, 6-dicarboxylate N-succinyltransferase activity.
(12) The aforementioned *Escherichia coli,* wherein the *dapC* gene is a DNA defined in the following (c) or (d):
   (c) a DNA comprising the nucleotide sequence of SEQ ID NO: 3, or
   (d) a DNA which is able to hybride with the nucleotide sequence of SEQ ID NO: 3 or a probe which can be prepared from the nucleotide sequence under stringent conditions, and coding for a protein having the succinyldiaminopimelate transaminase activity.
(13) The aforementioned *Escherichia coli,* wherein the *dapE* gene is a DNA defined in the following (e) or (f):
   (e) a DNA comprising the nucleotide sequence of SEQ ID NO: 5, or
   (f) a DNA which is able to hybridize with the nucleotide sequence of SEQ ID NO: 5 or a probe which can be prepared from the nucleotide sequence under stringent conditions, and coding for a protein having the succinyldiaminopimelate desuccinylase activity.
(14) The aforementioned *Escherichia coli,* wherein the *dapF* gene is a DNA defined in the following (g) or (h):
   (g) a DNA comprising the nucleotide sequence of SEQ ID NO: 7, or
   (h) a DNA which is able to hybridize with the nucleotide sequence of SEQ ID NO: 7 or a probe which can be prepared from the nucleotide sequence under stringent conditions, and coding for a protein having the diaminopimelate epimerase activity.
(15) The aforementioned *Escherichia coli,* wherein the diaminopimelate dehydrogenase is a protein defined in the following (I) or (J):
   (I) a protein having the amino acid sequence of SEQ ID NO: 10, 12, 14, 16, or 18,
   (J) a protein having the amino acid sequence of SEQ ID NO: 10, 12, 14, 16, or 18 including substitutions, deletions, insertions, or additions of one or several amino acid residues, and having the diaminopimelate dehydrogenase activity.
(16) The aforementioned *Escherichia coli,* wherein the ddh gene is a DNA defined in the following (i) or (j):
   (i) a DNA comprising the nucleotide sequence of SEQ ID NO: 9, 11, 13, 15, or 17, or
   (j) a DNA which is able to hybridize with the nucleotide sequence of SEQ ID NO: 9, 11, 13, 15, or 17 or a probe which can be prepared from the nucleotide sequence under stringent conditions, and coding for a protein having the diaminopimelate dehydrogenase activity.
(17) The aforementioned *Escherichia coli,* which further has a dihydrodipicolinate synthase which is desensitized to feedback inhibition by L-lysine, and an aspartokinase which is desensitized to feedback inhibition by L-lysine, and has enhanced activity of dihydrodipicolinate reductase.
(18) A method for producing L-lysine, which comprises culturing the aforementioned *Escherichia coli* in a medium and collecting L-lysine from the medium.

### Best Mode for Carrying out the Invention

Hereinafter, the present invention will be explained in detail.

### <1> Escherichia coli of the present invention

The *Escherichia coli* of the present invention (henceforth also referred to as the "bacterium of the present invention") is an *Escherichia coli* having an L-lysine-producing ability, which has been modified to decrease activity or activities of one or more enzymes of the meso-α,ε-diaminopimelic acid synthesis pathway, and into which a gene coding for diaminopimelate dehydrogenase has been introduced.

In addition to the aforementioned characteristics, the bacterium of the present invention preferably has a dihydrodipicolinate synthase which is desensitized to feedback inhibition by L-lysine, and an aspartokinase which is desensitized to feedback inhibition by L-lysine, and has an enhanced activity of dihydrodipicolinate reductase.

The bacterium of the present invention can be obtained by using *Escherichia coli* having L-lysine-producing ability as a parent strain, modifying it to decrease an activity of an enzyme belonging to the meso-DAP synthesis pathway, and further introducing a gene coding for diaminopimelate dehydrogenase. The order of the modification for decreasing an activity of an enzyme belonging to the meso-DAP synthesis pathway, and the introduction of a gene coding for diaminopimelate dehydrogenase is not particularly limited. Moreover, the L-lysine-producing ability may be imparted between or after the modification and the gene introduction.

The bacterium as a parent strain of *Escherichia coli* used for obtaining the bacterium of the present invention is not particularly limited. However, specific examples include, for example, those described in the work of Neidhardt et al. (Neidhardt F.C. et al., Escherichia coli and Salmonella typhimurium, American Society for Microbiology, Washington D.C., 1029, table 1). Specific examples include the *Escherichia coli* W3110 (ATCC 27325), *Escherichia coli* MG1655 (ATCC 47076) and so forth derived from the prototype wild-type strain, K12 strain.

These strains are available from, for example, American Type Culture Collection (Address: 12301 Parklawn Drive, Rockville, Maryland 20852, United States of America). That is, registration numbers are given to each of the strains, and the strains can be ordered using these registration numbers. The registration numbers of the strains are listed in the catalogue of the American Type Culture Collection.

### <1-1> Impartation of L-lysine-producing ability and Escherichia coli having L-lysine-producing ability

Examples of L-lysine-producing *Escherichia coli* bacteria include mutants having resistance to an L-lysine analogue. L-Lysine analogues inhibit growth of *Escherichia coli*, but this inhibition is fully or partially desensitized when L-lysine is present in the medium. Examples of the L-lysine analogues include, but are not limited to, oxalysine, lysine hydroxamate, S-(2-aminoethyl)-L-cysteine (AEC), γ-methyllysine, α-chlorocaprolactam and so forth. Mutants having resistance to these lysine analogues can be obtained by subjecting *Escherichia coli* to a conventional artificial mutagenesis. Specific examples of bacterial strains useful for producing L-lysine include *Escherichia coli* AJ11442 (FERM BP-1543, NRRL B-12185; see U.S. Patent No. 4,346,170) and *Escherichia coli* VL611. In these microorganisms, aspartokinase is desensitized to the feedback inhibition by L-lysine.

The WC196 strain may be used as an L-lysine-producing bacterium of *Escherichia coli.* This bacterial strain was bred by conferring AEC resistance to the W3110 strain, which was derived from *Escherichia coli* K-12. The resulting strain was designated *Escherichia coli* AJ13069 strain and was deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6, 1994 and assigned an accession number of FERM P-14690. Then, the deposit was converted to an international deposit under the provisions of the Budapest Treaty on September 29, 1995, and assigned an accession number of FERM BP-5252 (U.S. Patent No. 5,827,698).

Examples of parent strains which can be used to derive L-lysine-producing bacteria also include strains in which expression of one or more genes encoding an L-lysine biosynthetic enzyme is increased. Examples of such genes include, but are not limited to, genes coding for dihydrodipicolinate synthase (*dapA*), aspartokinase (*lysC*), dihydrodipicolinate reductase (*dapB*), diaminopimelate decarboxylase (*lysA*), phosphoenolpyrvate carboxylase (ppc), aspartate semialdehyde dehydrogenease (*asd*), and aspartase (*aspA*) (EP 1253195 A). In addition, the parent strains may have an increased level of expression of the gene involved in energy efficiency (*cyo*) (EP 1170376 A), the gene coding for nicotinamide nucleotide transhydrogenase (*pntAB*) (U.S. Patent No. 5,830,716), the ybjE gene (WO2005/073390), the gene coding for glutamate dehydrogenase (*gdhA*, Gene, 23:199-209 (1983)), or a combination thereof. Abbreviations for the genes of the enzymes are shown in the parentheses.

The nucleotide sequence of the *lysC* gene of *Escherichia coli* is shown in SEQ ID NO: 21, and the encoded amino acid sequence of aspartokinase is shown in SEQ ID NO: 22. The nucleotide sequence of the *dapA* gene of *Escherichia coli* is shown in SEQ ID NO: 23, and the encoded amino acid sequence of dihydrodipicolinate synthase is shown in SEQ ID NO: 24. Furthermore, the nucleotide sequence of the *dapB* gene of *Escherichia coli* is shown in SEQ ID NO: 25, and the encoded amino acid sequence of dihydrodipicolinate reductase is shown in SEQ ID NO: 26.

It is known that the wild-type dihydrodipicolinate synthase derived from *Escherichia coli* is subject to feedback inhibition by L-lysine, and it is known that the wild-type aspartokinase derived from *Escherichia coli* is subject to suppression and feedback inhibition by L-lysine. Therefore, when the *dapA* and *lysC* genes are used, these genes are preferably genes coding for mutant enzymes which are desensitized to the feedback inhibition by L-lysine.

Examples of DNAs encoding a mutant dihydrodipicolinate synthetase which is desensitized to feedback inhibition by L-lysine include a DNA encoding a protein which has the amino acid sequence of SEQ ID NO: 24 in which the histidine residue at position 118 is replaced by tyrosine residue. Examples of DNA encoding a mutant aspartokinase which is desensitized to feedback inhibition by L-lysine include a DNA encoding an AKIII having the amino acid sequence of SEQ ID NO: 22 in which the threonine residue at position 352, the glycine residue at position 323, and the methionine residue at position 318 are replaced by isoleucine, asparagine and isoleucine residues, respectively (U.S. Patent No. 5,661,012 and U.S. Patent No. 6,040,160). Such mutant DNAs can be obtained by site-specific mutagenesis using PCR or the like.

Wide host-range plasmids RSFD80, pCAB1, and pCABD2 derived from RSF1010 are known as plasmids containing a mutant *dapA* gene encoding a mutant *Escherichia coli* dihydrodipicolinate synthase and a mutant *lysC* gene encoding a mutant *Escherichia coli* aspartokinase (U.S. Patent No. 6,040,160). The *Escherichia coli* JM109 strain transformed with RSFD80 was named AJ12396 (U.S. Patent No. 6,040,160), and the strain was deposited at the National Institute of Bioscience and Human-Technology, Agency of Industrial Science and Technology, Ministry of International Trade and Industry (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on October 28, 1993 and assigned an accession number of FERM P-13936, and the deposit was then converted to an international deposit under the provisions of Budapest Treaty on November 1, 1994 and assigned an accession number of FERM BP-4859. RSFD80 can be obtained from the AJ12396 strain by a conventional method. pCAB1 was prepared by further inserting the *dapB* gene of *Escherichia coli* into RSFD80 described above. Furthermore, pCABD2 was prepared by further inserting the ddh gene of *Brevibacterium lactofermentum* (*Corynebacterium glutamicum*) 2256 strain (ATCC 13869) into pCAB1 described above (U.S. Patent No. 6,040,160).

Examples of L-lysine-producing bacteria or parent strains for deriving them also include strains in which activity of an enzyme that catalyzes a reaction which branches off from the L-lysine biosynthesis pathway and produces a compound other than L-lysine is decreased or made deficient. Examples of such enzymes that catalyze a reaction which branches off from the L-lysine biosynthesis pathway and produces a compound other than L-lysine include homoserine dehydrogenase, lysine decarboxylase (U.S. Patent No. 5,827,698), and malic enzyme (WO2005/010175). It is preferable that expressions of both the *cadA* and *ldcC* genes encoding lysine decarboxylase are decreased in order to decrease or delete the lysine decarboxylase activity (WO2006/038695).

In the bacterium used for the present invention, in order to enhance glycerol assimilability, expression of the *glpR* gene (EP 1715056) may be attenuated, or expression of glycerol metabolism genes (EP 1715055 A) such as *glpA, glpB, glpC, glpD, glpE, glpF, glpG, glpK, glpQ, glpT, glpX, tpiA, gldA, dhaK, dhaL, dhaM, dhaR, fsa* and *talC* genes may be enhanced.

### <1-2> Construction of Escherichia coli of the present invention

Hereinafter, the modification for reducing activity of an enzyme belonging to the meso-DAP synthesis pathway and introduction of a gene coding for diaminopimelate dehydrogenase will be explained.

The meso-DAP synthesis pathway of *Escherichia coli* is a pathway for generating meso-DAP (meso-2,6-diaminopimalate, meso-α,ε-diaminopimelate, or meso-diaminoheptanedioate) from (S)-2,3,4,5-tetrahydropyridine-2,6-dicarboxylate ((S)-2,3,4,5-tetrahydrodipicolinate), and is catalyzed by the enzymes for the following four step-reactions. These reactions are reversible reactions. In *Escherichia coli,* the meso-DAP synthesis pathway is also called DapDCEF pathway.

### 1) DapD (2,3,4,5-tetrahydropyridine-2,6-dicarboxylate N-succinyltransferase, EC 2.3.1.117)

### Succinyl-CoA + (S)-2,3,4,5-tetrahydropyridine-2,6-dicarboxylate + H₂O -> CoA + N-succinyl-L-2-amino-6-oxoheptanedioate

DapD is encoded by the *dapD* gene. The sequence of the *dapD* gene of *Escherichia coli* is shown in SEQ ID NO: 1, and the amino acid sequence of DapD is shown in SEQ ID NO: 2.

The enzymatic activity of DapD can be measured by referring to the method of S.A, Simms et al. (J. Biol. Chem., 1984, Mar 10; 259(5):2734-2791).

### 2) DapC (succinyldiaminopimelate transaminase, also referred to as SDAP aminotransferase, EC 2.6.1.17)

### N-Succinyl-LL-2,6-diaminoheptanedioate + 2-oxoglutarate -> N-succinyl-L-2-amino-6-oxoheptanedioate + L-glutamate

DapC is encoded by the *dapC* gene. The sequence of the *dapC* gene of *Escherichia coli* is shown in SEQ ID NO: 3, and the amino acid sequence is shown in SEQ ID NO: 4.

The enzymatic activity of DapC can be measured by the method of Thilo, M. et al. (J. Bacteriol., 2000, Jul; 182 (13) :3626-3631).

### 3) DapE (succinyldiaminopimelate desuccinylase, also referred to as SDAP desuccinylation enzyme, EC 3.5.1.18)

N-Succinyl-LL-2,6-diaminoheptanedioate + H₂O -> succinate + LL-2,6-diaminoheptanedioate

DapE is encoded by the *dapE* gene. The sequence of the *dapE* gene of *Escherichia coli* is shown in SEQ ID NO: 5, and the amino acid sequence is shown in SEQ ID NO: 6.

The enzymatic activity of DapE can be measured by the method of Lin, Y.K. et al. (J. Biol. Chem., 1988, Feb 5; 263(4):1622-1627).

### 4) DapF (diaminopimelate epimerase, EC 5.1.1.7)

### LL-2,6-Diaminoheptanedioate -> meso-2,6-diaminopimalate

DapF is encoded by the *dapF* gene. The sequence of the *dapF* gene of *Escherichia coli* is shown in SEQ ID NO: 7, and the amino acid sequence is shown in SEQ ID NO: 8.

The enzymatic activity of DapF can be measured by referring to the method of Wiseman, J.S. et al. (J. Biol. Chem., 1984, Jul 25; 259(14):8907-8914).

Furthermore, in the present invention, DDH (diaminopimelate dehydrogenase, EC 1.4.1.16) is an enzyme which reversibly generates (S)-2,3,4,5-tetrahydropyridine-2,6-dicarboxylate from meso-2,6-diaminopimalate, and catalyzes the following reaction.

### Meso-2,6-diaminopimalate + H₂O + NADP⁺ -> (S)-2, 3, 4, 5-tetrahydropyridine-2,6-dicarboxylate + NH₃ + NADPH + H⁺

The enzymatic activity of DDH can be measured by referring to the method of Misono, H. et al. (J. Biol. Chem., 255, 10599-10605, 1980).

Although *Escherichia* bacteria do not have any *ddh* gene coding for DDH, a *ddh* gene of a coryneform bacterium such as those of *Corynebacterium glutamicum* (SEQ ID NO: 9), *Brevibacterium lactofermentum* (SEQ ID NO: 11), and *Corynebacterium efficiens* (SEQ ID NO: 13) can be used.

The ddh gene of *Corynebacterium glutamicum* ATCC 13032 (NCg12528) is registered as Genbank NP_601818.2. GI:23308957, and the *ddh* gene of *Corynebacterium efficiens* (CE2498) is registered as NP_739108.1. GI:25029054.

Besides those of coryneform bacteria, the *ddh* gene of *Herminiimonas arsenicoxydans* (SEQ ID NO: 15), and the *ddh* gene of *Bacteroides thetaiotaomicron* (SEQ ID NO: 17) can be used. The *ddh* gene of *Herminiimonas arsenicoxydans* is registered as Genbank YP_001100730.1 GI:134095655, and the *ddh* gene of *Bacteroides thetaiotaomicron* is registered as NP_810892.1. GI:29347389.

The aforementioned genes and the aforementioned L-lysine biosynthesis enzyme genes are not limited to genes exactly corresponding to the gene information described above or genes having known sequences, and may be genes having a conservative mutation such as homologues thereof and artificially modified genes, so long as functions of the encoded proteins are not impaired. That is, the genes may be a gene coding for an amino acid sequence of a known protein including substitutions, deletions, insertions, additions or the like of one or several amino acid residues at one or several positions.

Although number meant by the term "one or several" used herein may differ depending on positions in the three-dimensional structure of the protein or types of amino acid residues, specifically, it may be preferably 1 to 20, more preferably 1 to 10, still more preferably 1 to 5. The conservative mutation is typically a conservative substitution. The conservative substitution is a substitution wherein substitution takes place mutually among Phe, Trp and Tyr, if the substitution site is an aromatic amino acid; among Leu, Ile and Val, if the substitution site is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having hydroxyl group. Specific examples of substitution considered conservative substitution include: substitution of Ser or Thr for Ala; substitution of Gln, His or Lys for Arg; substitution of Glu, Gln, Lys, His or Asp for Asn; substitution of Asn, Glu or Gln for Asp; substitution of Ser or Ala for Cys; substitution of Asn, Glu, Lys, His, Asp or Arg for Gln; substitution of Gly, Asn, Gln, Lys or Asp for Glu; substitution of Pro for Gly; substitution of Asn, Lys, Gln, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution of Trp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val. The mutation for such substitution, deletion, insertion, addition, inversion or the like of amino acid residues as described above also includes a naturally occurring mutation based on individual difference, difference in species of microorganisms from which the genes are derived (mutant or variant) and so forth. Such a gene can be obtained by modifying a nucleotide sequence of a known gene by, for example, site-specific mutagenesis, so that substitution, deletion, insertion or addition of an amino acid residue or residues is included at a specific site of the encoded protein.

Furthermore, a gene having the aforementioned conservative mutation may be a gene coding for a protein showing a homology of 80% or more, preferably 90% or more, more preferably 95% or more, particularly preferably 97% or more, to the entire sequence of the encoded protein, and having a function equivalent to that of a corresponding wild-type protein. In this specification, the term "homology" may be used to refer to "identity". Furthermore, codons of the gene sequences may be replaced with those which are easily used by a host into which the genes are introduced.

Genes having a conservative mutation may be those obtained by a method usually used for mutagenesis such as treatment with a mutagen.

Moreover, the genes may also be a DNA hybridizable with a probe that can be prepared from a known gene sequence, for example, the aforementioned gene sequences and sequences complementary to them, under stringent conditions, and coding for a protein having a function equivalent to that of a corresponding known gene product. The "stringent conditions" referred to conditions where a so-called specific hybrid is formed, and non-specific hybrid is not formed. Examples of the stringent conditions include, for example, conditions under which DNAs showing high homology to each other, for example, DNAs showing a homology of, for example, not less than 80%, preferably not less than 90%, more preferably not less than 95%, particularly preferably not less than 97%, hybridize with each other, and DNAs having homology lower than the above level do not hybridize with each other, and conditions of washing in ordinary Southern hybridization, i.e., conditions of washing once, preferably twice or three times, at salt concentrations and temperature of 1 x SSC, 0.1% SDS at 60°C, preferably 0.1 x SSC, 0.1% SDS at 60°C, more preferably 0.1 x SSC, 0.1% SDS at 68°C.

As the probe, a part of complementary sequences of the genes may also be used. Such a probe can be produced by PCR using oligonucleotides prepared on the basis of a known gene sequence as primers and a DNA fragment including nucleotide sequence of the gene as the template. When a DNA fragment having a length of about 300 bp is used as the probe, the washing conditions after hybridization under the aforementioned conditions can be exemplified by 2 x SSC, 0.1% SDS at 50°C.

The expression "to be modified so that an enzymatic activity of the meso-DAP synthesis pathway decreases" means to be modified so that activity of an enzyme belonging to the meso-DAP synthesis pathway (henceforth also referred to as "DapDCEF pathway"), specifically, at least one of the four enzymes, DapD, DapC, DapE, and DapF, is completely eliminated or decreased as compared to that of a non-modified strain of *Escherichia coli,* for example, a wild-type strain.

The enzyme of which activity is decreased may be any of DapD, DapC, DapE, and DapF, and may consist of two or more kinds of them. It is preferable to decrease an activity of an enzyme functioning in the upstream region of the DapDCEF pathway, and it is particularly preferable to perform modification so that at least the activity of DapD decreases.

Decrease of an enzymatic activity of the DapDCEF pathway is preferably means that, for example, each enzymatic activity in the DapDCEF pathway is decreased to 50% or less, preferably 30% or less, more preferably 10% or less, per cell, as compared to that of a non-modified strain, for example, a wild-type strain.

Examples of strain as an object of the comparison include the *Escherichia coli* W3110 (ATCC 27325), *Escherichia coli* MG1655 (ATCC 47076) and so forth, derived from the prototype wild-type strain, K12 strain, as wild-type strain.

Such modification that an enzymatic activity in the DapDCEF pathway decreases is attained by, specifically, deleting a part of or entire gene on a chromosome coding for an enzyme of the DapDCEF pathway, specifically, the coding region of the *dapD, dapC, dapE,* or *dapF* gene, or modifying an expression control sequence such as promoter and Shine-Dalgarno (SD) sequence. Furthermore, expression of a gene can also be decreased by modification of a non-translation region other than expression control sequence. Furthermore, the entire gene including the sequences on both sides of the gene on a chromosome may be deleted. Furthermore, it can also be attained by introduction of a mutation for an amino acid substitution (missense mutation), a stop codon (nonsense mutation), or a frame shift mutation which adds or deletes one or two nucleotides, into a coding region coding for an enzyme on a chromosome (Journal of Biological Chemistry, 272:8611-8617 (1997); Proceedings of the National Academy of Sciences, USA, 95 5511-5515 (1998); Journal of Biological Chemistry, 266, 20833-20839 (1991)).

In the present invention, it is preferable to decrease an intracellular enzymatic activity by deleting a part of or entire expression regulatory sequence of the gene on a chromosome such as a promoter region, or a part of or entire coding region or a non-coding region, or inserting another sequences into one of these sequences using homologous recombination. However, the modification may be a modification caused by a usual mutagenesis based on X-ray or ultraviolet irradiation or use of a mutagene such as N-methyl-N'-nitro-N-nitrosoguanidine, so long as the modification decreases an enzymatic activity of the DapDCEF pathway.

Modification of an expression control sequence is preferably performed for one or more nucleotides, more preferably two or more nucleotides, particularly preferably three or more nucleotides. When deletion is performed for a coding region, the region to be deleted may be an N-terminal region, an internal region or a C-terminal region, or even the entire coding region, so long as the function of the enzyme protein to be produced is decreased or deleted. Deletion of a longer region can usually more surely inactivate a gene. Furthermore, it is preferred that reading frames locating upstream and downstream of the region to be deleted are not the same.

When another sequence is inserted into a coding region, the sequence may be inserted into any region of the gene, and insertion of a longer sequence can usually more surely inactivate the gene coding for an enzyme. It is preferred that reading frames locating upstream and downstream of the insertion site are not the same. The other sequence is not particularly limited so long as a sequence which decreases or deletes function of the enzyme protein is chosen, and examples include, for example, a transposon carrying an antibiotic resistance gene or a gene useful for L-lysine production, and so forth.

A gene on a chromosome can be modified as described above by, for example, preparing a deletion-type version of the gene in which a partial sequence of the gene is deleted so that the deletion-type gene does not produce a protein which do not normally function, and transforming a bacterium with a DNA containing the deletion-type gene to cause homologous recombination between the deletion-type gene and the native gene on the chromosome, and thereby substitute the deletion-type gene for the gene on the chromosome. The protein encoded by the deletion-type gene has a conformation different from that of the wild-type enzyme protein, if it is even produced, and thus the function is decreased or deleted. Such gene disruption based on gene substitution utilizing homologous recombination has been already established, and there are a method called Red-driven integration (Datsenko, K.A, and Wanner, B.L., Proc. Natl. Acad. Sci. USA, 97:6640-6645 (2000)), a method of using a linear DNA such as a method utilizing the Red driven integration in combination with an excisive system derived from λ phage (Cho, E.H., Gumport, R.I., Gardner, J.F., J. Bacteriol., 184:5200-5203 (2002)), a method of using a plasmid containing a temperature sensitive replication origin or a plasmid capable of conjugative transfer, a method of utilizing a suicide vector not having replication origin in a host (U.S. Patent No. 6,303,383, Japanese Patent Laid-open No. 05-007491), and so forth.

Decrease of expression amount of a gene can be confirmed by comparing the amount of mRNA transcribed from the gene with that in a wild-type or non-modified strain. The expression amount can be confirmed by Northern hybridization, RT-PCR (Molecular Cloning, Cold Spring Harbor Laboratory Press, Cold Spring Harbor, USA, 2001)), and so forth.

Decrease of amount of a protein encoded by a gene can be confirmed by Western blotting using antibodies (Molecular Cloning, Cold spring Harbor Laboratory Press, Cold Spring Harbor, USA, 2001).

In order to introduce a gene coding for DDH (*ddh*) into *Escherichia coli,* for example, *Escherichia coli* may be transformed with a *ddh* gene by using a vector such as a plasmid and a phage. Examples of such vectors include pUC19, pUC18, pBR322, pHSG299, pHSG298, pHSG399, pHSG398, RSF1010, pMW119, pMW118, pMW219, pMW218, and so forth. Although an inherent promoter of the DDH gene may be used, so long as the gene can be expressed in *Escherichia coli* with it, a promoter which efficiently functions in *Escherichia coli* may also be used. Examples of such promoters include lac promoter, trp promoter, trc promoter, tac promoter, PR promoter and PL promoter of λ phage, tet promoter, and so forth.

The ddh gene may also be incorporated into the chromosome of *Escherichia coli* by a method using transduction, a transposon (Berg, D.E. and Berg, C.M., Bio/Technol., 1, 417 (1983)), Mu phage (Japanese Patent Laid-open No. 2-109985), or homologous recombination (Experiments in Molecular Genetics, Cold Spring Harbor Lab. (1972)). Furthermore, copy number of the *ddh* gene may be increased by transferring the *ddh* genes incorporated into the chromosome.

Incorporation of the *ddh* gene can be confirmed by, for example, Southern hybridization. Furthermore, whether *Escherichia coli* into which the *ddh* gene has been introduced has the DDH activity or not can be confirmed by, for example, measuring the DDH activity according to the method described in Haruo Misono, Fermentation and Industry, 45, 964 (1987). Moreover, DDH can also be detected by Western blotting using an antibody.

### <2> Method for producing L-lysine

The method for producing L-lysine of the present invention comprises culturing the bacterium of the present invention in a medium to produce and accumulate L-lysine in the medium or cells, and collecting L-lysine from the medium or cells.

As the medium to be used, media conventionally used in the production of L-lysine by fermentation using microorganisms can be used. That is, conventional media containing a carbon source, a nitrogen source, inorganic ions, and optionally other organic components as required may be used. As the carbon source, saccharides such as glucose, sucrose, lactose, galactose, fructose, and starch hydrolysate; alcohols such as glycerol and sorbitol; and organic acids such as fumaric acid, citric acid and succinic acid can be used. As the nitrogen source, inorganic ammonium salts such as ammonium sulfate, ammonium chloride, and ammonium phosphate, organic nitrogen such as soybean hydrolysate, ammonia gas, aqueous ammonia, and so forth may be used. As for organic trace nutrient sources, it is desirable that the medium contains required substances such as vitamin B₁ and L-homoserine, yeast extract or the like in appropriate amounts. Other than the above, potassium phosphate, magnesium sulfate, iron ions, manganese ions and so forth are added in small amounts, as required. In addition, the medium used in the present invention may be either a natural medium or synthetic medium, so long as a medium containing a carbon source, a nitrogen source, and inorganic ions, and containing other organic trace components as required is used.

In the present invention, glycerol is particularly preferably used as a carbon source. Although glycerol may be a reagent glycerol, it is desirable to use industrially produced glycerol containing impurities. For example, it is desirable to use glycerol industrially produced by the esterification reaction for biodiesel fuel production (Mu Y, et al, Biotechnol Lett., 28, 1755-91759 (2006); Haas M.J., et al., Bioresour. Technol., 97, 4, 671-8678 (2006)).

Glycerol contained in the medium used in the present invention may be a sole carbon source, or a mixed medium to which other carbon sources are added in addition to glycerol may also be used. Preferable other carbon sources are saccharides such as glucose, fructose, sucrose, lactose, galactose, blackstrap molasses, starch hydrolysate, sugar solution obtained by hydrolysis of biomass, alcohols such as ethanol, and organic acids such as fumaric acid, citric acid, and succinic acid. When a mixed medium is used, it is desirable that glycerol is contained in the medium at a ratio of 50% or more, preferably 60% or more, more preferably 70% or more, still more preferably 80% or more, especially preferably 90% or more, based on the total carbon source contained in the medium.

The culture is preferably performed for 1 to 7 days under aerobic conditions. The culture temperature is preferably 24 to 37°C, and pH during the culture is preferably between 5 to 9. To adjust pH, inorganic or organic acidic, or alkaline substances, ammonia gas, and so forth can be used. To collect L-lysine from the fermentation medium, a combination of known methods can be used, such as by using an ion exchange resin and precipitation. When L-lysine accumulates in the cells, supersonic waves, for example, or the like can be used to disrupt cells, and L-lysine can be collected by using an ion exchange resin or the like, from the supernatant obtained by removing the cells from the cell-disrupted suspension by centrifugation.

The production may also be performed by a method in which fermentation is performed by controlling pH of the medium during culture to be 6.5 to 9.0 and pH of the medium at the end of the culture to be 7.2 to 9.0 and controlling the pressure in the fermentation tank during fermentation to be positive, or supplying carbon dioxide or a mixed gas containing carbon dioxide to the medium so that bicarbonate ions and/or carbonate ions are present in the culture medium in an amount of at least 2 g/L during the culture, and these bicarbonate ions and/or carbonate ions serve as counter ions of cations mainly consisting of basic amino acid, and lysine is then collected (refer to Japanese Patent Laid-open No. 2002-065287, U.S. Published Patent Application No. 2002/025564).

### Examples

Hereinafter, the present invention will be still more specifically explained with reference to examples.

### Example 1: Construction of dapD-disrupted L-lysine-producing bacterium

### <1-1> Construction of dapD gene-disrupted strain

First, a *dapD*-disrupted strain was constructed using the *Escherichia coli* wild-type strain, the MG1655 strain.

Using pMW118 (λattL-Km^{r}-λattR) plasmid (refer to International Patent Publication WO2006/093322) as the template, and synthetic oligonucleotides of SEQ ID NOS: 19 and 20 having sequences corresponding to both ends of the sequences of the attachment sites of λ phage, *attL* and *attR*, at the 3' ends and sequences corresponding to parts of the *dapD* gene as the target gene at the 5' ends as primers, PCR was performed to construct MG1655ΔdapD::att-Km strain according to the λ-red method described in U.S. Patent Published Application No. 2006/0160191 and WO2005/010175. A Km-resistant recombinant was obtained according to the λ-red method by culturing the bacterium at 37°C on an L-agar medium containing Km (kanamycin, 50 mg/L) as plate culture and selecting a Km-resistant recombinant.

### <1-2> Transduction of L-lysine-producing bacterium WC196LC/pCABD2 strain with ΔdapD::att-kan

P1 lysate was obtained from the MG1655ΔdapD::att-Km strain obtained in <1-1> in a conventional manner, and the L-lysine-producing bacterium WC196ΔcadAΔldcC/pCABD2 strain constructed by the method described in U.S. Patent Published Application No. 2006/0160191 was used as a host to construct WC196ΔcadAΔldcChdapD::att-Km/pCABD2 strain according to the P1 transduction method. The WC196ΔcadAAldc strain was obtained from the *Escherichia coli* WC196 strain by disrupting lysine decarboxylase genes, the *cadA* and *ldc* according to the method using the Red-driven integration method (Datsenko K.A., Wanner, B.L., 2000, Proc. Nat1. Acad. Sci. USA, 97, 6640-6645) and the excision system derived from λ-phage (Cho, E.H., Gumport, R.I., Gardner, J.F., J. Bacteriol., 184:5200-5203 (2002)) in combination (refer to WO2005/010175). A strain obtained by introducing pCABD2 into this strain is the WC196ΔcadAΔldcC/pCABD2 strain.

The objective transduced strain was obtained by performing plate culture at 37°C on an L-agar medium containing Km (kanamycin, 50 mg/L) and Sm (streptomycin, 20 mg/L), and selecting a Km-resistant and Sm-resistant recombinant strain.

Furthermore, these strains were each cultured at 37°C in an L medium containing 20 mg/L of streptomycin until final OD600 reached about 0.6, then the culture was added with an equal volume of 40% glycerol solution, and the mixture was stirred, divided into appropriate volumes, and stored at -80°C. These mixtures will be called glycerol stocks.

### Example 2: Evaluation of L-lysine producing ability of dapD-disrupted L-lysine-producing bacterium

The glycerol stocks of the strains obtained in Example 1 were thawed, and uniformly applied to an L-plate containing 20 mg/L of streptomycin in a volume of 100 µL each, and culture was performed at 37°C for 24 hours. The cells in an amount of about 1/8 of the obtained cells on one plate were suspended in 0.5 mL of physiological saline, and turbidity of the suspension was measured at 600 nm using a spectrophotometer (U-2000, Hitachi). The suspension containing the obtained cells was inoculated into 20 mL of a fermentation medium (MS medium, composition is shown below) containing 20 mg/L of streptomycin in a 500-mL Sakaguchi flask in such a volume that turbidity of the mixture became 0.15 at 600 nm, and culture was performed at 37°C and 114 rpm for 24 hours on a reciprocally shaking culture machine. After the culture, amounts of accumulated L-lysine and remained glucose in the medium were measured using Biotec Analyzer AS210 (SAKURA SEIKI). Glycerol accumulated in the medium was also measured using Biotec Analyzer BF-5 (Oji Scientific Instruments).

| | |
|---|---|
| Composition of fermentation medium, g/L Glycerol or glucose | 40 |
| (NH₄)2SO₄ | 24 |
| KH₂PO₄ | 11.0 |
| MgSO₄ • 7H₂O | 1.0 |
| FeSO₄ • 7H₂O | 0.01 |
| MnSO₄ • 5H₂O | 0.01 |
| Yeast Extract | 2.0 |

The medium was adjusted to pH 7.0 with KOH, autoclaved at 115°C for 10 minutes (provided that glycerol or glucose and MgSO₄•7H₂O were separately sterilized) and then added with 30 g/L of CaCO₃ (Japanese Pharmacopoeia) (subjected to hot air sterilization at 180°C for 2 hours). Streptomycin was added in an amount of 20 mg/L.

The results are shown in Table 1 (OD means cell amount indicated in terms of absorbance at 660 nm measured for culture diluted 26 times, Lys (g/L) means amount of L-lysine accumulated in the flask, Glucose (g/L) and Glycerol (g/L) mean amounts of glucose and glycerol remained in the medium, respectively, and Yield (%) means L-lysine yield based on the substrate). As seen from the results shown in Table 1, the WC196ΔcadAΔldcCΔdapD::att-Km/pCABD2 strain accumulated L-lysine in a larger amount as compared to that obtained with the WC196ΔcadAΔldcC/pCABD2 strain in which *dapD* gene was not disrupted.

**Table 1**

| MS glucose medium | | | | |
|---|---|---|---|---|
| Strain | O.D. (x26) | Lys (g/L) | Glucose (g/L) | Yield (%) |
| WC196ΔcadAΔldcC/pCABD2 strain | 0.361 | 8.28 | 18.67 | 38.82 |
| WC196ΔcadAΔldcCAdapD::att-Km/pCABD2 strain | 0.377 | 8.75 | 18.13 | 40.01 |

| MS glycerol medium | | | | |
|---|---|---|---|---|
| Strain | O.D. (x26) | Lys (g/L) | Glycerol (g/L) | Yield (%) |
| WC196ΔcadAΔldcC/pCABD2 strain | 0.248 | 2.79 | 28.64 | 24.57 |
| WC196ΔcadAΔldcCΔdapD::att-Km/pCABD2 strain | 0.320 | 5.10 | 23.69 | 31.12 |

### Explanation of Sequence Listing

SEQ ID NO: 1: Nucleotide sequence of *E. coli dapD*
SEQ ID NO: 2: Amino acid sequence of *E. coli* DapD
SEQ ID NO: 3: Nucleotide sequence of *E. coli dapC*
SEQ ID NO: 4: Amino acid sequence of *E. coli* DapC
SEQ ID NO: 5: Nucleotide sequence of *E. coli dapE*
SEQ ID NO: 6: Amino acid sequence of *E. coli* DapE
SEQ ID NO: 7: Nucleotide sequence of *E. coli dapF*
SEQ ID NO: 8: Amino acid sequence of *E. coli* DapF
SEQ ID NO: 9: Nucleotide sequence of *ddh* gene of *C. glutamicum*
SEQ ID NO: 10: Amino acid sequence of DDH of *C. glutamicum*
SEQ ID NO: 11: Nucleotide sequence of *ddh* gene of *B. lactofermentum*
SEQ ID NO: 12: Amino acid sequence of DDH of *B. lactofermentum*
SEQ ID NO: 13: Nucleotide sequence of *ddh* gene of *C. efficiens*
SEQ ID NO: 14: Amino acid sequence of DDH of *C. efficiens*
SEQ ID NO: 15: Nucleotide sequence of *ddh* gene of *H. arsenicoxydans*
SEQ ID NO: 16: Amino acid sequence of DDH of *H. arsenicoxydans*
SEQ ID NO: 17: Nucleotide sequence of *ddh* gene of *B. thetaiotaomicron*
SEQ ID NO: 18: Amino acid sequence of DDH of *B. thetaiotaomicron*
SEQ ID NO 19: Primer for deletion of *dapD* gene
SEQ ID NO: 20: Primer for deletion of *dapD* gene
SEQ ID NO: 21: Nucleotide sequence of *E. coli lysc*
SEQ ID NO: 22: Amino acid sequence of *E. coli* LysC
SEQ 10 NO: 23: Nucleotide sequence of *E. coli dapA*
SEQ ID NO: 24: Amino acid sequence of *E. coli* DapA
SEQ ID NO: 25: Nucleotide sequence of *E. coli dapB*
SEQ ID NO: 26: Amino acid sequence of *E. coli* DapB

### Industrial Applicability

According to the present invention, in L-lysine production by fermentation using *Escherichia coli,* production amount and/or fermentation yield of L-lysine can be improved. Moreover, the present invention can be used for breeding of L-lysine-producing bacterium of *Escherichia coli.*

## Claims

1. A method for producing L-lysine, which comprises culturing in a medium an *Escherichia coli* having L-lysine-producing ability and collecting L-lysine from the medium,
wherein the *Escherichia coli* has been modified to decrease activity or activities of one or more kinds of enzymes of the meso-α,ε-diaminopimelic acid synthesis pathway, and
wherein a gene coding for diaminopimelate dehydrogenase has been introduced into the *Escherichia coli.*

2. The method according to claim 1, wherein the enzymes of the meso-α,ε-diaminopimelic acid synthesis pathway are selected from 2,3,4,5-tetrahydropyridine-2,6-dicarboxylate N-succinyltransferase, succinyldiaminopimelate transaminase, succinyldiaminopimelate desuccinylase, and diaminopimelate epimerase.

3. The method according to claim 2, wherein the 2,3,4,5-tetrahydropyridine-2,6-dicarboxylate
N-succinyltransferase, succinyldiaminopimelate transaminase, succinyldiaminopimelate desuccinylase, and diaminopimelate epimerase are encoded by the *dapD* gene, *dapC* gene, *dapE* gene, and *dapF* gene, respectively.

4. The method according to claim 3, wherein the activities of the enzymes of the meso-α,ε-diaminopimelic acid synthesis pathway are decreased by decreasing expression of the genes or by disrupting the genes.

5. The method according to any one of claims 2 to 4, wherein the *Escherichia coli* has been modified to decrease at least the activity of 2,3,4,5-tetrahydropyridine-2,6-dicarboxylate N-succinyltransferase.

6. The method according to any one of claims 1 to 5, wherein the gene coding for diaminopimelate dehydrogenase is the ddh gene of a coryneform bacterium.

7. The method according to any one of claims 2 to 6, wherein the 2,3,4,5-tetrahydropyridine-2,6-dicarboxylate N-succinyltransferase is a protein defined in the following (A) or (B):
(A) a protein having the amino acid sequence of SEQ ID NO: 2,
(B) a protein having the amino acid sequence of SEQ ID NO: 2 including substitutions, deletions, insertions, or additions of one or several amino acid residues, and having the 2,3,4,5-tetrahydropyridine-2,6-dicarboxylate N-succinyltransferase activity.

8. The method according to any one of claims 2 to 7, wherein the succinyldiaminopimelate transaminase is a protein defined in the following (C) or (D):
(C) a protein having the amino acid sequence of SEQ ID NO: 4,
(D) a protein having the amino acid sequence of SEQ ID NO: 4 including substitutions, deletions, insertions, or additions of one or several amino acid residues, and having the succinyldiaminopimelate transaminase activity.

9. The method according to any one of claims 2 to 8, wherein the succinyldiaminopimelate desuccinylase is a protein defined in the following (E) or (F):
(E) a protein having the amino acid sequence of SEQ ID NO: 6,
(F) a protein having the amino acid sequence of SEQ ID NO: 6 including substitutions, deletions, insertions, or additions of one or several amino acid residues, and having the succinyldiaminopimelate desuccinylase activity.

10. The method according to any one of claims 2 to 9, wherein the diaminopimelate epimerase is a protein defined in the following (G) or (H):
(G) a protein having the amino acid sequence of SEQ ID NO: 8,
(H) a protein having the amino acid sequence of SEQ ID NO: 8 including substitutions, deletions, insertions, or additions of one or several amino acid residues, and having the diaminopimelate epimerase activity.

11. The method according to any one of claims 3 to 10, wherein the *dapD* gene is a DNA defined in the following (a) or (b):
(a) a DNA comprising the nucleotide sequence of SEQ ID NO: 1, or
(b) a DNA which is able to hybridize with the nucleotide sequence of SEQ ID NO: 1 or a probe which can be prepared from the nucleotide sequence under stringent conditions, and coding for a protein having the 2,3,4,5-tetrahydropyridine-2,6-dicarboxylate N-succinyltransferase activity.

12. The method according to any one of claims 3 to 11, wherein the *dapC* gene is a DNA defined in the following (c) or (d):
(c) a DNA comprising the nucleotide sequence of SEQ ID NO: 3, or
(d) a DNA which is able to hybridize with the nucleotide sequence of SEQ ID NO: 3 or a probe which can be prepared from the nucleotide sequence under stringent conditions, and coding for a protein having the succinyldiaminopimelate transaminase activity.

13. The method according to any one of claims 3 to 12, wherein the *dapE* gene is a DNA defined in the following (e) or (f):
(e) a DNA comprising the nucleotide sequence of SEQ ID NO: 5, or
(f) a DNA which is able to hybridize with the nucleotide sequence of SEQ ID NO: 5 or a probe which can be prepared from the nucleotide sequence under stringent conditions, and coding for a protein having the succinyldiaminopimelate desuccinylase activity.

14. The method according to any one of claims 3 to 13, wherein the *dapF* gene is a DNA defined in the following (g) or (h):
(g) a DNA comprising the nucleotide sequence of SEQ ID NO: 7, or
(h) a DNA which is able to hybridize with the nucleotide sequence of SEQ ID NO: 7 or a probe which can be prepared from the nucleotide sequence under stringent conditions, and coding for a protein having the diaminopimelate epimerase activity.

15. The method according to any one of claims 1 to 14, wherein the diaminopimelate dehydrogenase is a protein defined in the following (I) or (J):
(I) a protein having the amino acid sequence of SEQ ID NO: 10, 12, 14, 16, or 18,
(J) a protein having the amino acid sequence of SEQ ID NO: 10, 12, 14, 16, or 18 including substitutions, deletions, insertions, or additions of one or several amino acid residues, and having the diaminopimelate dehydrogenase activity.

16. The method according to any one of claims 6 to 13, wherein the ddh gene is a DNA defined in the following (i) or (j):
(i) a DNA comprising the nucleotide sequence of SEQ ID NO: 9, 11, 13, 15, or 17, or
(j) a DNA which is able to hybridize with the nucleotide sequence of SEQ ID NO: 9, 11, 13, 15, or 17 or a probe which can be prepared from the nucleotide sequence under stringent conditions, and coding for a protein having the diaminopimelate dehydrogenase activity.

17. The method according to any one of claims 1 to 16, wherein the *Escherichia coli* further has a dihydrodipicolinate synthase which is desensitized to feedback inhibition by L-lysine, and an aspartokinase which is desensitized to feedback inhibition by L-lysine, and has enhanced activity of dihydrodipicolinate reductase.

## Patentansprüche

1. Verfahren zur Herstellung von L-Lysin, welches die Kultivierung eines Escherichia coli mit der Fähigkeit, L-Lysin herzustellen, in einem Medium und das Gewinnen von L-Lysin aus dem Medium umfasst,
wobei das Escherichia coli modifiziert wurde, um die Aktivität oder die Aktivitäten einer oder mehrerer Arten von Enzymen des meso-α, ε-Diaminopimelinsäure-Synthesewegs zu verringern, und
wobei ein Gen, das für Diaminopimelat-Dehydrogenase kodiert, in das Escherichia coli eingebracht wurde.

2. Verfahren gemäß Anspruch 1, wobei die Enzyme des meso-α, ε-Diaminopimelinsäure-Synthesewegs ausgewählt sind aus 2,3,4,5-Tetrahydropyridin-2,6-dicarboxylat-N-succinyltransferase, Succinyldiaminopimelat-Transaminase, Succinyldiaminopimelat-Desuccinylase und Diaminopimelat-Epimerase.

3. Verfahren gemäß Anspruch 2, wobei die 2,3,4,5-Tetrahydropyridin-2,6-dicarboxylat-N-succinyltransferase, Succinyldiaminopimelat-Transaminase, Succinyldiaminopimelat-Desuccinylase und Diaminopimelat-Epimerase durch das dapD-Gen, das dapC-Gen, das dapE-Gen bzw. das dapF-Gen kodiert werden.

4. Verfahren gemäß Anspruch 3, wobei die Aktivitäten der Enzyme des meso-α, ε-Diaminopimelinsäure-Synthesewegs verringert werden, indem die Expression der Gene verringert wird, oder indem die Gene zerstört werden.

5. Verfahren gemäß mindestens einem der Ansprüche 2 bis 4, wobei das Escherichia coli modifiziert wurde, um mindestens die Aktivität der 2,3,4,5-Tetrahydropyridin-2,6-dicarboxylat-N-succinyltransferase zu verringern.

6. Verfahren gemäß mindestens einem der Ansprüche 1 bis 5, wobei das Gen, das für die Diaminopimelat-Dehydrogenase kodiert, das ddh-Gen eines koryneformen Bakteriums ist.

7. Verfahren gemäß mindestens einem der Ansprüche 2 bis 6, wobei die 2,3,4,5-Tetrahydropyridin-2,6-dicarboxylat-N-succinyltransferase ein Protein ist, das in den folgenden (A) oder (B) definiert ist:
(A) ein Protein mit der Aminosäuresequenz von SEQ ID NO: 2,
(B) ein Protein mit der Aminosäuresequenz von SEQ ID NO: 2 einschließlich Substitutionen, Deletionen, Insertionen oder Additionen eines oder mehrerer Aminosäurereste, und mit der 2,3,4,5-Tetrahydropydridin-2,6-dicarboxylat-N-succinyltransferase-Aktivität.

8. Verfahren gemäß mindestens einem der Ansprüche 2 bis 7, wobei die Succinyldiaminopimelat-Transaminase ein Protein ist, das in den folgenden (C) oder (D) definiert ist:
(C) ein Protein mit der Aminosäuresequenz von SEQ ID NO: 4,
(D) ein Protein mit der Aminosäuresequenz von SEQ ID NO: 4 einschließlich Substitutionen, Deletionen, Insertionen oder Additionen eines oder mehrerer Aminosäurereste, und mit der Succinyldiaminopimelat-Transaminase-Aktivität.

9. Verfahren gemäß mindestens einem der Ansprüche 2 bis 8, wobei die Succinyldiaminopimelat-Desuccinylase ein Protein ist, das in den folgenden (E) oder (F) definiert ist:
(E) ein Protein mit der Aminosäuresequenz von SEQ ID NO: 6,
(F) ein Protein mit der Aminosäuresequenz von SEQ ID NO: 6 einschließlich Substitutionen, Deletionen, Insertionen oder Additionen eines oder mehrerer Aminosäurereste, und mit der Succinyldiaminopimelat-Desuccinylase-Aktivität.

10. Verfahren gemäß mindestens einem der Ansprüche 2 bis 9, wobei die Diaminopimelat-Epimerase ein Protein ist, das in den folgenden (G) oder (H) definiert ist:
(G) ein Protein mit der Aminosäuresequenz von SEQ ID NO: 8,
(H) ein Protein mit der Aminosäuresequenz von SEQ ID NO: 8 einschließlich Substitutionen, Deletionen, Insertionen oder Additionen eines oder mehrerer Aminosäurereste, und mit der Diaminopimelat-Epimerase-Aktivität.

11. Verfahren gemäß mindestens einem der Ansprüche 3 bis 10, wobei das dapD-Gen eine DNA ist, die in den folgenden (a) oder (b) definiert ist:
(a) eine DNA, die die Nukleotidsequenz von SEQ ID NO: 1 umfasst oder
(b) eine DNA, die in der Lage ist, mit der Nukleotidsequenz von SEQ ID NO: 1 oder einer Sonde, die aus der Nukleotidsequenz hergestellt werden kann, unter stringenten Bedingungen zu hybridisieren, und die für ein Protein mit der 2,3,4,5-Tetrahydropyridin-2,6-dicarboxylat-N-succinyltransferase-Aktivität kodiert.

12. Verfahren gemäß mindestens einem der Ansprüche 3 bis 11, wobei das dapC-Gen eine DNA ist, die in den folgenden (c) oder (d) definiert ist:
(c) eine DNA, die die Nukleotidsequenz von SEQ ID NO: 3 umfasst, oder
(d) eine DNA, die in der Lage ist, mit der Nukleotidsequenz von SEQ ID NO: 3 oder einer Sonde, die aus der Nukleotidsequenz hergestellt werden kann, unter stringenten Bedingungen zu hybridisieren, und die für ein Protein mit der Succinyldiaminopimelat-Transaminase-Aktivität kodiert.

13. Verfahren gemäß mindestens einem der Ansprüche 3 bis 12, wobei das dapE-Gen eine DNA ist, die in den folgenden (e) oder (f) definiert ist:
(e) eine DNA, die die Nukleotidsequenz von SEQ ID NO: 5 umfasst, oder
(f) eine DNA, die in der Lage ist, mit der Nukleotidsequenz von SEQ ID NO: 5 oder einer Sonde, die aus der Nukleotidsequenz hergestellt werden kann, unter stringenten Bedingungen zu hybridisieren, und die für ein Protein mit der Succinyldiaminopimelat-Desuccinylase-Aktivität kodiert.

14. Verfahren gemäß mindestens einem der Ansprüche 3 bis 13, wobei das dapF-Gen eine DNA ist, die in den folgenden (g) oder (h) definiert ist:
(g) eine DNA, die die Nukleotidsequenz von SEQ ID NO: 7 umfasst, oder
(h) eine DNA, die in der Lage ist, mit der Nukleotidsequenz von SEQ ID NO: 7 oder einer Sonde, die aus der Nukleotidsequenz hergestellt werden kann, unter stringenten Bedingungen zu hybridisieren, und die für ein Protein mit der Diaminopimelat-Epimerase-Aktivität kodiert.

15. Verfahren gemäß mindestens einem der Ansprüche 1 bis 14, wobei die Diaminopimelat-Dehydrogenase ein Protein ist, das in den folgenden (I) oder (J) definiert ist:
(I) ein Protein mit der Aminosäuresequenz von SEQ ID NO: 10, 12, 14, 16 oder 18,
(J) ein Protein mit der Aminosäuresequenz von SEQ ID NO: 10, 12, 14, 16 oder 18 einschließlich Substitutionen, Deletionen, Insertionen oder Additionen eines oder mehrerer Aminosäurereste, und mit der Diaminopimelat-Dehydrogenase-Aktivität.

16. Verfahren gemäß mindestens einem der Ansprüche 6 bis 13, wobei das ddh-Gen eine DNA ist, die in den folgenden (i) oder (j) definiert ist:
(i) eine DNA, die die Nukleotidsequenz von SEQ ID NO: 9, 11, 13, 15 oder 17 umfasst, oder
(j) eine DNA, die in der Lage ist, mit der Nukleotidsequenz von SEQ ID NO: 9, 11, 13, 15 oder 17 oder einer Sonde, die aus der Nukleotidsequenz hergestellt werden kann, unter stringenten Bedingungen zu hybridisieren, und die für ein Protein mit der Diaminopimelat-Dehydrogenase-Aktivität kodiert.

17. Verfahren gemäß mindestens einem der Ansprüche 1 bis 16, wobei das Escherichia coli ferner eine Dihydrodipicolinat-Synthase besitzt, die gegen Feedback-Hemmung durch L-Lysin desensibilisiert ist, und eine Aspartokinase besitzt, die gegen Feedback-Hemmung durch L-Lysin desensibilisiert ist, und eine verstärkte Aktivität der Dihydrodipicolinat-Reduktase besitzt.

## Revendications

1. Procédé de production de L-lysine, qui comprend la culture dans un milieu d'une Escherichia coli ayant une capacité de production de la L-lysine et la collecte de la L-lysine du milieu,
dans lequel l'Escherichia coli a été modifiée pour diminuer l'activité ou les activités d'un ou de plusieurs types d'enzymes de la voie de synthèse de l'acide méso-α, ε-diaminopimélique, et
dans lequel un gène codant pour la diaminopimélate déshydrogénase a été introduit dans l'Escherichia coli.

2. Procédé selon la revendication 1, dans lequel les enzymes de la voie de synthèse de l'acide méso-α, ε-diaminopimélique sont choisies parmi la 2,3,4,5-tétrahydropyridine-2,6-dicarboxylate N-succinyltransférase, la succinyldiaminopimélate transaminase, la succinyldiaminopimélate désuccinylase, et la diaminopimélate épimérase.

3. Procédé selon la revendication 2, dans lequel la 2,3,4,5-tétrahydropyridine-2,6-dicarboxylate N-succinyltransférase, la succinyldiaminopimélate transaminase, la succinyldiaminopimélate désuccinylase, la diaminopimélate épimérase sont codées par le gène dapD, le gène dapC, le gène dapE , et le gène dapF, respectivement.

4. Procédé selon la revendication 3, dans lequel les activités des enzymes de la voie de synthèse de l'acide méso-α, ε-diaminopimélique sont diminuées en diminuant l'expression des gènes ou en perturbant les gènes.

5. Procédé selon l'une quelconque des revendications 2 à 4, dans lequel l'Escherichia coli a été modifiée pour diminuer au moins l'activité de la 2,3,4,5-tétrahydropyridine-2,6-dicarboxylate N-succinyltransférase.

6. Procédé selon l'une quelconque des revendications 1 à 5, dans lequel
le gène codant pour la diaminopimélate déshydrogénase est le gène ddh d'une bactérie corynéforme.

7. Procédé selon l'une quelconque des revendications 2 à 6, dans lequel la 2,3,4,5-tétrahydropyridine-2,6-dicarboxylate N-succinyltransférase est une protéine définie dans les protéines (A) ou (B) suivantes :
(A) une protéine ayant la séquence d'acides aminés de SEQ ID NO:2,
(B) une protéine ayant la séquence d'acides aminés de SEQ ID NO:2 comportant des substitutions, des suppressions, des insertions ou des additions d'un ou de plusieurs résidus d'acides aminés, et ayant l'activité de la 2,3,4,5-tétrahydropyridine-2,6-dicarboxylate N-succinyltransférase.

8. Procédé selon l'une quelconque des revendications 2 à 7, dans lequel la succinyldiaminopimélate transaminase est une protéine définie dans les protéines (C) ou (D) suivantes :
(C) une protéine ayant la séquence d'acides aminés de SEQ ID NO:4,
(D) une protéine ayant la séquence d'acides aminés de SEQ ID NO:4 comportant des substitutions, des suppressions, des insertions ou des additions d'un ou de plusieurs résidus d'acides aminés, et ayant l'activité de la succinyldiaminopimélate transaminase.

9. Procédé selon l'une quelconque des revendications 2 à 8, dans lequel la succinyldiaminopimélate désuccinylase est une protéine définie dans les protéines (E) ou (F) suivantes :
(E) une protéine ayant la séquence d'acides aminés de SEQ ID NO:6,
(F) une protéine ayant la séquence d'acides aminés de SEQ ID NO:6 comportant des substitutions, des suppressions, des insertions ou des additions d'un ou de plusieurs résidus d'acides aminés, et ayant l'activité de la succinyldiaminopimélate désuccinylase.

10. Procédé selon l'une quelconque des revendications 2 à 9, dans lequel la diaminopimélate épimérase est une protéine définie dans les protéines (G) ou (H) suivantes :
(G) une protéine ayant la séquence d'acides aminés de SEQ ID NO:8,
(H) une protéine ayant la séquence d'acides aminés de SEQ ID NO:8 comportant des substitutions, des suppressions, des insertions ou des additions d'un ou de plusieurs résidus d'acides aminés, et ayant l'activité de la diaminopimélate épimérase.

11. Procédé selon l'une quelconque des revendications 3 à 10, dans lequel le gène dapD est un ADN défini dans les ADNs (a) ou (b) suivantes :
(a) un ADN comprenant la séquence nucléotidique de SEQ ID NO:1, ou
(b) un ADN qui est capable de s'hybrider avec la séquence nucléotidique de SEQ ID NO:1 ou une sonde qui peut être préparée à partir de la séquence nucléotidique dans des conditions stringentes, et codant pour une protéine ayant l'activité de la 2,3,4,5-tétrahydropyridine-2,6-dicarboxylate N-succinyltransférase.

12. Procédé selon l'une quelconque des revendications 3 à 11, dans lequel le gène dapC est un ADN défini dans les ADNs (c) ou (d) suivants :
(c) un ADN comprenant la séquence nucléotidique de SEQ ID NO:3, ou
(d) un ADN qui est capable de s'hybrider avec la séquence nucléotidique de SEQ ID NO:3 ou une sonde qui peut être préparée à partir de la séquence nucléotidique dans des conditions stringentes, et codant pour une protéine ayant l'activité de la succinyldiaminopimélate transaminase.

13. Procédé selon l'une quelconque des revendications 3 à 12, dans lequel le gène dapE est un ADN défini dans les ADNs (e) ou (f) suivants :
(e) un ADN comprenant la séquence nucléotidique de SEQ ID NO:5, ou
(f) un ADN qui est capable de s'hybrider avec la séquence nucléotidique de SEQ ID NO:5 ou une sonde qui peut être préparée à partir de la séquence nucléotidique dans des conditions stringentes, et codant pour une protéine ayant l'activité de la succinyldiaminopimélate désuccinylase.

14. Procédé selon l'une quelconque des revendications 3 à 13, dans lequel le gène dapF est un ADN défini dans les ADNs (g) ou (h) suivants :
(g) un ADN comprenant la séquence nucléotidique de SEQ ID NO:7, ou
(h) un ADN qui est capable de s'hybrider avec la séquence nucléotidique de SEQ ID NO:7 ou une sonde qui peut être préparée à partir de la séquence nucléotidique dans des conditions stringentes, et codant pour une protéine ayant l'activité de la diaminopimélate épimérase.

15. Procédé selon l'une quelconque des revendications 1 à 14, dans lequel la diaminopimélate déshydrogénase est une protéine définie dans les protéines (I) ou (J) suivantes:
(I) une protéine ayant la séquence d'acides aminés de SEQ ID NO:10, 12, 14, 16, ou 18,
(J) une protéine ayant la séquence d'acides aminés de SEQ ID NO:10, 12, 14, 16, ou 18 comportant des substitutions, des suppressions, des insertions ou des additions d'un ou de plusieurs résidus d'acides aminés, et ayant l'activité de la diaminopimélate déshydrogénase.

16. Procédé selon l'une quelconque des revendications 6 à 13, dans lequel le gène ddh est un ADN défini dans les ADNs (i) ou (j) suivants :
(i) un ADN comprenant la séquence nucléotidique de SEQ ID NO:9, 11, 13, 15, ou 17, ou
(j) un ADN qui est capable de s'hybrider avec la séquence nucléotidique de SEQ ID NO:9, 11, 13, 15, ou 17 ou une sonde qui peut être préparée à partir de la séquence nucléotidique dans des conditions stringentes, et codant pour une protéine ayant l'activité de la diaminopimélate déshydrogénase.

17. Procédé selon l'une quelconque des revendications 1 à 16, dans lequel l'Escherichia coli possède en outre une dihydrodipicolinate synthase qui est désensibilisée à la rétro-inhibition par la L-lysine, et une aspartokinase qui est désensibilisée à la rétro-inhibition par la L-lysine, et a une activité améliorée de la dihydrodipicolinate réductase.


### SEQUENCE LISTING

<110> Ajinomoto Co., Inc.
<120> A method for producing L-lysine
<130> C919-C8129
<150> JP2007-190795
   <151> 2007-07-23
<160> 26
<170> Patent In version 3.5
<210> 1
   <211> 825
   <212> DNA
   <213> Escherichia coli
<220>
   <221> CDS
   <222> (1)..(825)
<400> 1
   atg cag cag tta cag aac att att gaa acc gct ttt gaa cgc cgt gcc      48
   Met Gln Gln Leu Gln Asn Ile Ile Glu Thr Ala Phe Glu Arg Arg Ala
   1               5                   10                  15
   gag atc acg cca gcc aat gca gac acc gtt acc cgc gaa gcg gta aat      96
   Glu Ile Thr Pro Ala Asn Ala Asp Thr Val Thr Arg Glu Ala Val Asn
               20                  25                  30
   cag gtg atc gcc ctg ctg gat tcc ggc gca ctg cgt gta gcg gaa aaa     144
   Gln Val Ile Ala Leu Leu Asp Ser Gly Ala Leu Arg Val Ala Glu Lys
           35                  40                  45
   att gac ggt cag tgg gtg acg cat cag tgg ttg aaa aaa gcg gtg ctg     192
   Ile Asp Gly Gln Trp Val Thr His Gln Trp Leu Lys Lys Ala Val Leu
       50                  55                  60
   ctc tct ttc cgt att aat gat aat cag gtg atc gaa ggg gca gaa agc     240
   Leu Ser Phe Arg Ile Asn Asp Asn Gln Val Ile Glu Gly Ala Glu Ser
   65                  70                  75                  80
   cgc tac ttc gac aaa gtg ccg atg aaa ttc gcc gac tac gac gaa gca     288
   Arg Tyr Phe Asp Lys Val Pro Met Lys Phe Ala Asp Tyr Asp Glu Ala
                   85                  90                  95
   cgt ttc cag aaa gaa ggc ttc cgc gtt gtg cca cca gcg gcg gta cgt     336
   Arg Phe Gln Lys Glu Gly Phe Arg Val Val Pro Pro Ala Ala Val Arg
               100                 105                 110
   cag ggt gcg ttt att gcc cgt aac acc gtg ctg atg ccg tct tac gtc     384
   Gln Gly Ala Phe Ile Ala Arg Asn Thr Val Leu Met Pro Ser Tyr Val
           115                 120                 125
   aac atc ggc gca tat gtt gat gaa ggc acc atg gtt gat acc tgg gcg     432
   Asn Ile Gly Ala Tyr Val Asp Glu Gly Thr Met Val Asp Thr Trp Ala
       130                 135                 140
   acc gtc ggt tct tgt gcg cag att ggt aaa aac gtc cac ctt tcc ggt     480
   Thr Val Gly Ser Cys Ala Gln Ile Gly Lys Asn Val His Leu Ser Gly
   145                 150                 155                 160
   ggc gtg ggc atc ggc ggc gtg ctg gaa ccg ctg cag gct aac cca acc     528
   Gly Val Gly Ile Gly Gly Val Leu Glu Pro Leu Gln Ala Asn Pro Thr
                   165                 170                 175
   atc att gaa gat aat tgc ttc atc ggc gcg cgc tct gaa gtg gtt gaa     576
   Ile Ile Glu Asp Asn Cys Phe Ile Gly Ala Arg Ser Glu Val Val Glu
               180                 185                 190
   ggg gtg att gtc gaa gaa ggt tcc gtc att tcc atg ggc gta tac att       624
   Gly Val Ile Val Glu Glu Gly Ser Val Ile Ser Met Gly Val Tyr Ile
           195                 200                 205
   ggt cag agc acc cgt att tac gac cgt gaa acc ggc gaa atc cac tac       672
   Gly Gln Ser Thr Arg Ile Tyr Asp Arg Glu Thr Gly Glu Ile His Tyr
       210                 215                 220
   ggt cgc gtt ccg gcg ggg tct gtg gtt gtt tca ggt aat ctg ccg tca       720
   Gly Arg Val Pro Ala Gly Ser Val Val Val Ser Gly Asn Leu Pro Ser
   225                 230                 235                 240
   aaa gat ggc aaa tac agc ctc tac tgt gcg gtt atc gtt aag aaa gtt       768
   Lys Asp Gly Lys Tyr Ser Leu Tyr Cys Ala Val Ile Val Lys Lys Val
                   245                 250                 255
   gac gcg aaa act cgc ggc aaa gtc ggc att aac gaa ctg ctg cgt acc       816
   Asp Ala Lys Thr Arg Gly Lys Val Gly Ile Asn Glu Leu Leu Arg Thr
               260                 265                 270
   atc gac taa                                                           825
   Ile Asp
<210> 2
   <211> 274
   <212> PRT
   <213> Escherichia coli
<400> 2
   Met Gln Gln Leu Gln Asn Ile Ile Glu Thr Ala Phe Glu Arg Arg Ala
   1               5                   10                  15
   Glu Ile Thr Pro Ala Asn Ala Asp Thr Val Thr Arg Glu Ala Val Asn
               20                  25                  30
   Gln Val Ile Ala Leu Leu Asp Ser Gly Ala Leu Arg Val Ala Glu Lys
           35                  40                  45
   Ile Asp Gly Gln Trp Val Thr His Gln Trp Leu Lys Lys Ala Val Leu
       50                  55                  60
   Leu Ser Phe Arg Ile Asn Asp Asn Gln Val Ile Glu Gly Ala Glu Ser
   65                  70                  75                  80
   Arg Tyr Phe Asp Lys Val Pro Met Lys Phe Ala Asp Tyr Asp Glu Ala
                   85                  90                  95
   Arg Phe Gln Lys Glu Gly Phe Arg Val Val Pro Pro Ala Ala Val Arg
               100                 105                 110
   Gln Gly Ala Phe Ile Ala Arg Asn Thr Val Leu Met Pro Ser Tyr Val
           115                 120                 125
   Asn Ile Gly Ala Tyr Val Asp Glu Gly Thr Met Val Asp Thr Trp Ala
       130                 135                 140
   Thr Val Gly Ser Cys Ala Gln Ile Gly Lys Asn Val His Leu Ser Gly
   145                 150                 155                 160
   Gly Val Gly Ile Gly Gly Val Leu Glu Pro Leu Gln Ala Asn Pro Thr
                   165                 170                 175
   Ile Ile Glu Asp Asn Cys Phe Ile Gly Ala Arg Ser Glu Val Val Glu
               180                 185                 190
   Gly Val Ile Val Glu Glu Gly Ser Val Ile Ser Met Gly Val Tyr Ile
           195                 200                 205
   Gly Gln Ser Thr Arg Ile Tyr Asp Arg Glu Thr Gly Glu Ile His Tyr
       210                 215                 220
   Gly Arg Val Pro Ala Gly Ser Val Val Val Ser Gly Asn Leu Pro Ser
   225                 230                 235                 240
   Lys Asp Gly Lys Tyr Ser Leu Tyr Cys Ala Val Ile Val Lys Lys Val
                   245                 250                 255
   Asp Ala Lys Thr Arg Gly Lys Val Gly Ile Asn Glu Leu Leu Arg Thr
               260                 265                 270
   Ile Asp
<210> 3
   <211> 1221
   <212> DNA
   <213> Escherichia coli
<220>
   <221> CDS
   <222> (1)..(1221)
<400> 3
   atg gca att gaa caa aca gca att aca cgc gcg act ttc gat gaa gtg       48
   Met Ala Ile Glu Gln Thr Ala Ile Thr Arg Ala Thr Phe Asp Glu Val
   1               5                   10                  15
   atc ctg ccg att tat gct ccg gca gag ttt att ccg gta aaa ggt cag       96
   Ile Leu Pro Ile Tyr Ala Pro Ala Glu Phe Ile Pro Val Lys Gly Gln
               20                  25                  30
   ggc agc cga atc tgg gat cag caa ggc aag gag tat gtc gat ttc gcg      144
   Gly Ser Arg Ile Trp Asp Gln Gln Gly Lys Glu Tyr Val Asp Phe Ala
           35                  40                  45
   ggt ggc att gca gtt acg gcg ttg ggc cat tgc cat cct gcg ctg gtg      192
   Gly Gly Ile Ala Val Thr Ala Leu Gly His Cys His Pro Ala Leu Val
       50                  55                  60
   aac gcg tta aaa acc cag ggc gaa act ctg tgg cat atc agt aac gtt      240
   Asn Ala Leu Lys Thr Gln Gly Glu Thr Leu Trp His Ile Ser Asn Val
   65                  70                  75                  80
   ttc acc aat gaa ccg gcg ctg cgt ctt ggg cgt aaa ctg att gag gca      288
   Phe Thr Asn Glu Pro Ala Leu Arg Leu Gly Arg Lys Leu Ile Glu Ala
                   85                  90                  95
   acg ttt gcc gaa cgc gtg gtg ttt atg aac tcc ggc acg gaa gct aac      336
   Thr Phe Ala Glu Arg Val Val Phe Met Asn Ser Gly Thr Glu Ala Asn
               100                 105                 110
   gaa acc gcc ttt aaa ctg gca cgc cat tac gcc tgt gtg cgt cat agc      384
   Glu Thr Ala Phe Lys Leu Ala Arg His Tyr Ala Cys Val Arg His Ser
           115                 120                 125
   ccg ttc aaa acc aaa att att gcc ttc cat aac gct ttt cat ggt cgc      432
   Pro Phe Lys Thr Lys Ile Ile Ala Phe His Asn Ala Phe His Gly Arg
       130                 135                 140
   tcg ctg ttt acc gtt tcg gtg ggt ggg cag cca aaa tat tcc gac ggc      480
   Ser Leu Phe Thr Val Ser Val Gly Gly Gln Pro Lys Tyr Ser Asp Gly
   145                 150                 155                 160
   ttt ggg ccg aaa ccg gca gac atc atc cac gtt ccc ttt aac gat ctc      528
   Phe Gly Pro Lys Pro Ala Asp Ile Ile His Val Pro Phe Asn Asp Leu
                   165                 170                 175
   cat gca gtg aaa gcg gtg atg gat gat cac acc tgt gcg gtg gtg gtt      576
   His Ala Val Lys Ala Val Met Asp Asp His Thr Cys Ala Val Val Val
               180                 185                 190
   gag ccg atc cag ggc gag ggc ggt gtg acg gca gcg acg cca gag ttt      624
   Glu Pro Ile Gln Gly Glu Gly Gly Val Thr Ala Ala Thr Pro Glu Phe
           195                 200                 205
   ttg cag ggc ttg cgc gag ctg tgc gat caa cat cag gca tta ttg gtg      672
   Leu Gln Gly Leu Arg Glu Leu Cys Asp Gln His Gln Ala Leu Leu Val
       210                 215                 220
   ttt gat gaa gtg cag tgc ggg atg ggg cgg acc ggc gat ttg ttt gct      720
   Phe Asp Glu Val Gln Cys Gly Met Gly Arg Thr Gly Asp Leu Phe Ala
   225                 230                 235                 240
   tac atg cac tac ggc gtt acg ccg gat att ctg acc tct gcg aaa gcg      768
   Tyr Met His Tyr Gly Val Thr Pro Asp Ile Leu Thr Ser Ala Lys Ala
                   245                 250                 255
   tta ggc ggc ggc ttc ccg att agc gcc atg ctg acc acg gcg gaa att      816
   Leu Gly Gly Gly Phe Pro Ile Ser Ala Met Leu Thr Thr Ala Glu Ile
               260                 265                 270
   gct tct gcg ttt cat cct ggt tct cac ggt tcc acc tac ggc ggt aat      864
   Ala Ser Ala Phe His Pro Gly Ser His Gly Ser Thr Tyr Gly Gly Asn
           275                 280                 285
   cct ctg gcc tgt gca gta gcg ggg gcg gcg ttt gat atc atc aat acc      912
   Pro Leu Ala Cys Ala Val Ala Gly Ala Ala Phe Asp Ile Ile Asn Thr
       290                 295                 300
   cct gaa gtg ctg gaa ggc att cag gcg aaa cgc cag cgt ttt gtt gac      960
   Pro Glu Val Leu Glu Gly Ile Gln Ala Lys Arg Gln Arg Phe Val Asp
   305                 310                 315                 320
   cat ctg cag aag atc gat cag cag tac gat gta ttt agc gat att cgc     1008
   His Leu Gln Lys Ile Asp Gln Gln Tyr Asp Val Phe Ser Asp Ile Arg
                   325                 330                 335
   ggt atg ggg ctg ttg att ggc gca gag ctg aaa cca cag tac aaa ggt     1056
   Gly Met Gly Leu Leu Ile Gly Ala Glu Leu Lys Pro Gln Tyr Lys Gly
               340                 345                 350
   cgg gcg cgt gat ttc ctg tat gcg ggc gca gag gct ggc gta atg gtg     1104
   Arg Ala Arg Asp Phe Leu Tyr Ala Gly Ala Glu Ala Gly Val Met Val
           355                 360                 365
   ctg aat gcc gga ccg gat gtg atg cgt ttt gca ccg tcg ctg gtg gtg     1152
   Leu Asn Ala Gly Pro Asp Val Met Arg Phe Ala Pro Ser Leu Val Val
       370                 375                 380
   gaa gat gcg gat atc gat gaa ggg atg caa cgt ttc gcc cac gcg gtg     1200
   Glu Asp Ala Asp Ile Asp Glu Gly Met Gln Arg Phe Ala His Ala Val
   385                 390                 395                 400
   gcg aag gtg gtt ggg gcg taa                                         1221
   Ala Lys Val Val Gly Ala
                   405
<210> 4
   <211> 406
   <212> PRT
   <213> Escherichia coli
<400> 4
   Met Ala Ile Glu Gln Thr Ala Ile Thr Arg Ala Thr Phe Asp Glu Val
   1               5                   10                  15
   Ile Leu Pro Ile Tyr Ala Pro Ala Glu Phe Ile Pro Val Lys Gly Gln
               20                  25                  30
   Gly Ser Arg Ile Trp Asp Gln Gln Gly Lys Glu Tyr Val Asp Phe Ala
           35                  40                  45
   Gly Gly Ile Ala Val Thr Ala Leu Gly His Cys His Pro Ala Leu Val
       50                  55                  60
   Asn Ala Leu Lys Thr Gln Gly Glu Thr Leu Trp His Ile Ser Asn Val
   65                  70                  75                  80
   Phe Thr Asn Glu Pro Ala Leu Arg Leu Gly Arg Lys Leu Ile Glu Ala
                   85                  90                  95
   Thr Phe Ala Glu Arg Val Val Phe Met Asn Ser Gly Thr Glu Ala Asn
               100                 105                 110
   Glu Thr Ala Phe Lys Leu Ala Arg His Tyr Ala Cys Val Arg His Ser
           115                 120                 125
   Pro Phe Lys Thr Lys Ile Ile Ala Phe His Asn Ala Phe His Gly Arg
       130                 135                 140
   Ser Leu Phe Thr Val Ser Val Gly Gly Gln Pro Lys Tyr Ser Asp Gly
   145                 150                 155                 160
   Phe Gly Pro Lys Pro Ala Asp Ile Ile His Val Pro Phe Asn Asp Leu
                   165                 170                 175
   His Ala Val Lys Ala Val Met Asp Asp His Thr Cys Ala Val Val Val
               180                 185                 190
   Glu Pro Ile Gln Gly Glu Gly Gly Val Thr Ala Ala Thr Pro Glu Phe
           195                 200                 205
   Leu Gln Gly Leu Arg Glu Leu Cys Asp Gln His Gln Ala Leu Leu Val
       210                 215                 220
   Phe Asp Glu Val Gln Cys Gly Met Gly Arg Thr Gly Asp Leu Phe Ala
   225                 230                 235                 240
   Tyr Met His Tyr Gly Val Thr Pro Asp Ile Leu Thr Ser Ala Lys Ala
                   245                 250                 255
   Leu Gly Gly Gly Phe Pro Ile Ser Ala Met Leu Thr Thr Ala Glu Ile
               260                 265                 270
   Ala Ser Ala Phe His Pro Gly Ser His Gly Ser Thr Tyr Gly Gly Asn
           275                 280                 285
   Pro Leu Ala Cys Ala Val Ala Gly Ala Ala Phe Asp Ile Ile Asn Thr
       290                 295                 300
   Pro Glu Val Leu Glu Gly Ile Gln Ala Lys Arg Gln Arg Phe Val Asp
   305                 310                 315                 320
   His Leu Gln Lys Ile Asp Gln Gln Tyr Asp Val Phe Ser Asp Ile Arg
                   325                 330                 335
   Gly Met Gly Leu Leu Ile Gly Ala Glu Leu Lys Pro Gln Tyr Lys Gly
               340                 345                 350
   Arg Ala Arg Asp Phe Leu Tyr Ala Gly Ala Glu Ala Gly Val Met Val
           355                 360                 365
   Leu Asn Ala Gly Pro Asp Val Met Arg Phe Ala Pro Ser Leu Val Val
       370                 375                 380
   Glu Asp Ala Asp Ile Asp Glu Gly Met Gln Arg Phe Ala His Ala Val
   385                 390                 395                 400
   Ala Lys Val Val Gly Ala
                   405
<210> 5
   <211> 1128
   <212> DNA
   <213> Escherichia coli
<220>
   <221> CDS
   <222> (1) .. (1128)
<400> 5
   atg tcg tgc ccg gtt att gag ctg aca caa cag ctt att cgc cgc cct          48
   Met Ser Cys Pro Val Ile Glu Leu Thr Gln Gln Leu Ile Arg Arg Pro
   1               5                   10                  15
   tcc ctg agt cct gat gat gca gga tgc cag gct ttg ttg att gaa cgt          96
   Ser Leu Ser Pro Asp Asp Ala Gly Cys Gln Ala Leu Leu Ile Glu Arg
               20                  25                  30
   ttg cag gcg atc ggt ttt acc gtt gaa cgc atg gac ttt gcc gat acg         144
   Leu Gln Ala Ile Gly Phe Thr Val Glu Arg Met Asp Phe Ala Asp Thr
           35                  40                  45
   cag aat ttt tgg gca tgg cgt ggg cag ggt gaa acg tta gcc ttt gcc         192
   Gln Asn Phe Trp Ala Trp Arg Gly Gln Gly Glu Thr Leu Ala Phe Ala
       50                  55                  60
   ggg cat acc gac gtg gtg ccg cct ggc gac gcc gat cgt tgg atc aat         240
   Gly His Thr Asp Val Val Pro Pro Gly Asp Ala Asp Arg Trp Ile Asn
   65                  70                  75                  80
   ccc ccg ttt gaa ccc acc att cgt gac ggc atg tta ttc ggg cgc ggt         288
   Pro Pro Phe Glu Pro Thr Ile Arg Asp Gly Met Leu Phe Gly Arg Gly
                   85                  90                  95
   gcg gca gat atg aaa ggc tcg ctg gcg gcg atg gtg gtg gcg gca gaa         336
   Ala Ala Asp Met Lys Gly Ser Leu Ala Ala Met Val Val Ala Ala Glu
               100                           105                           110
   cgt ttt gtc gca caa cat ccc aac cat acg ggg cga ctg gca ttt ctg        384
   Arg Phe Val Ala Gln His Pro Asn His Thr Gly Arg Leu Ala Phe Leu
           115                           120                           125
   atc acc tct gat gaa gaa gcc agt gcc cac aac ggt acg gta aaa gtc        432
   Ile Thr Ser Asp Glu Glu Ala Ser Ala His Asn Gly Thr Val Lys Val
       130                           135                           140
   gtc gaa gcg tta atg gca cgt aat gag cgt ctc gat tac tgc ctg gtt        480
   Val Glu Ala Leu Met Ala Arg Asn Glu Arg Leu Asp Tyr Cys Leu Val
   145                           150                           155                           160
   ggc gaa ccg tcg agt atc gaa gtg gta ggt gat gtg gtg aaa aat ggt        528
   Gly Glu Pro Ser Ser Ile Glu Val Val Gly Asp Val Val Lys Asn Gly
                   165                           170                           175
   cgt cgc gga tca tta acc tgc aac ctt acc att cat ggc gtt cag ggg        576
   Arg Arg Gly Ser Leu Thr Cys Asn Leu Thr Ile His Gly Val Gln Gly
               180                           185                           190
   cat gtt gcc tac cca cat ctg gct gac aat ccg gta cat cgc gca gca        624
   His Val Ala Tyr Pro His Leu Ala Asp Asn Pro Val His Arg Ala Ala
           195                           200                           205
   cct ttc ctt aat gaa tta gtg gct att gag tgg gat cag ggc aat gaa        672
   Pro Phe Leu Asn Glu Leu Val Ala Ile Glu Trp Asp Gln Gly Asn Glu
       210                           215                           220
   ttc ttc ccg gcg acc agt atg cag att gcc aat att cag gcg gga acg        720
   Phe Phe Pro Ala Thr Ser Met Gln Ile Ala Asn Ile Gln Ala Gly Thr
   225                           230                           235                           240
   ggc agt aac aac gtt att ccg ggt gaa ctg ttt gtg cag ttt aac ttc        768
   Gly Ser Asn Asn Val Ile Pro Gly Glu Leu Phe Val Gln Phe Asn Phe
                   245                           250                           255
   cgc ttc agc acc gaa ctg act gat gag atg atc aaa gcg cag gtg ctt        816
   Arg Phe Ser Thr Glu Leu Thr Asp Glu Met Ile Lys Ala Gln Val Leu
               260                           265                           270
   gcc ctg ctt gaa aaa cat caa ctg cgc tat acg gtg gat tgg tgg ctt        864
   Ala Leu Leu Glu Lys His Gln Leu Arg Tyr Thr Val Asp Trp Trp Leu
           275                           280                           285
   tcc ggg cag cca ttt ttg acc gcg cgc ggt aaa ctg gtg gat gcg gtc        912
   Ser Gly Gln Pro Phe Leu Thr Ala Arg Gly Lys Leu Val Asp Ala Val
       290                           295                           300
   gtt aac gcg gtt gag cac tat aat gaa att aaa ccg cag cta ctg acc        960
   Val Asn Ala Val Glu His Tyr Asn Glu Ile Lys Pro Gln Leu Leu Thr
   305                           310                           315                           320
   aca ggc gga acg tcc gac ggg cgc ttt att gcc cgc atg ggg gcg cag       1008
   Thr Gly Gly Thr Ser Asp Gly Arg Phe Ile Ala Arg Met Gly Ala Gln
                   325                           330                           335
   gtg gtg gaa ctc ggg ccg gtc aat gcc act att cat aaa att aat gaa       1056
   Val Val Glu Leu Gly Pro Val Asn Ala Thr Ile His Lys Ile Asn Glu
               340                           345                           350
   tgt gtg aac gct gcc gac ctg cag cta ctt gcc cgt atg tat caa cgt       1104
   Cys Val Asn Ala Ala Asp Leu Gln Leu Leu Ala Arg Met Tyr Gln Arg
           355                           360                           365
   atc atg gaa cag ctc gtc gcc tga                                       1128
   Ile Met Glu Gln Leu Val Ala
       370                           375
<210> 6
   <211> 375
   <212> PRT
   <213> Escherichia coli
<400> 6
   Met Ser Cys Pro Val Ile Glu Leu Thr Gln Gln Leu Ile Arg Arg Pro
   1 5 10 15
   Ser Leu Ser Pro Asp Asp Ala Gly Cys Gln Ala Leu Leu Ile Glu Arg
   20 25 30
   Leu Gln Ala Ile Gly Phe Thr Val Glu Arg Met Asp Phe Ala Asp Thr
   35 40 45
   Gln Asn Phe Trp Ala Trp Arg Gly Gln Gly Glu Thr Leu Ala Phe Ala
   50 55 60
   Gly His Thr Asp Val Val Pro Pro Gly Asp Ala Asp Arg Trp Ile Asn
   65 70 75 80
   Pro Pro Phe Glu Pro Thr Ile Arg Asp Gly Met Leu Phe Gly Arg Gly
   85 90 95
   Ala Ala Asp Met Lys Gly Ser Leu Ala Ala Met Val Val Ala Ala Glu
   100 105 110
   Arg Phe Val Ala Gln His Pro Asn His Thr Gly Arg Leu Ala Phe Leu
   115 120 125
   Ile Thr Ser Asp Glu Glu Ala Ser Ala His Asn Gly Thr Val Lys Val
   130 135 140
   Val Glu Ala Leu Met Ala Arg Asn Glu Arg Leu Asp Tyr Cys Leu Val
   145 150 155 160
   Gly Glu Pro Ser Ser Ile Glu Val Val Gly Asp Val Val Lys Asn Gly
   165 170 175
   Arg Arg Gly Ser Leu Thr Cys Asn Leu Thr Ile His Gly Val Gln Gly
   180 185 190
   His Val Ala Tyr Pro His Leu Ala Asp Asn Pro Val His Arg Ala Ala
   195 200 205
   Pro Phe Leu Asn Glu Leu Val Ala Ile Glu Trp Asp Gln Gly Asn Glu
   210 215 220
   Phe Phe Pro Ala Thr Ser Met Gln Ile Ala Asn Ile Gln Ala Gly Thr
   225 230 235 240
   Gly Ser Asn Asn Val Ile Pro Gly Glu Leu Phe Val Gln Phe Asn Phe
   245 250 255
   Arg Phe Ser Thr Glu Leu Thr Asp Glu Met Ile Lys Ala Gln Val Leu
   260 265 270
   Ala Leu Leu Glu Lys His Gln Leu Arg Tyr Thr Val Asp Trp Trp Leu
   275 280 285
   Ser Gly Gln Pro Phe Leu Thr Ala Arg Gly Lys Leu Val Asp Ala Val
   290 295 300
   Val Asn Ala Val Glu His Tyr Asn Glu Ile Lys Pro Gln Leu Leu Thr
   305 310 315 320
   Thr Gly Gly Thr Ser Asp Gly Arg Phe Ile Ala Arg Met Gly Ala Gln
   325 330 335
   Val Val Glu Leu Gly Pro Val Asn Ala Thr Ile His Lys Ile Asn Glu
   340 345 350
   Cys Val Asn Ala Ala Asp Leu Gln Leu Leu Ala Arg Met Tyr Gln Arg
   355 360 365
   Ile Met Glu Gln Leu Val Ala
   370 375
<210> 7
   <211> 825
   <212> DNA
   <213> Escherichia coli
<220>
   <221> CDS
   <222> (1)..(825)
<400> 7
   atg cag ttc tcg aaa atg cat ggc ctt ggc aac gat ttt atg gtc gtc      48
   Met Gln Phe Ser Lys Met His Gly Leu Gly Asn Asp Phe Met Val Val
   1               5                   10                  15
   gac gcg gta acg cag aat gtc ttt ttt tca ccg gag ctg att cgt cgc      96
   Asp Ala Val Thr Gln Asn Val Phe Phe Ser Pro Glu Leu Ile Arg Arg
               20                  25                  30
   ctg gct gat cgg cac ctg ggg gta ggg ttt gac caa ctg ctg gtg gtt     144
   Leu Ala Asp Arg His Leu Gly Val Gly Phe Asp Gln Leu Leu Val Val
           35                  40                  45
   gag ccg ccg tat gat cct gaa ctg gat ttt cac tat cgc att ttc aat     192
   Glu Pro Pro Tyr Asp Pro Glu Leu Asp Phe His Tyr Arg Ile Phe Asn
       50                  55                  60
   gct gat ggc agt gaa gtg gcg cag tgc ggc aac ggt gcg cgc tgc ttt     240
   Ala Asp Gly Ser Glu Val Ala Gln Cys Gly Asn Gly Ala Arg Cys Phe
   65                  70                  75                  80
   gcc cgt ttt gtg cgt ctg aaa gga ctg acc aat aag cgt gat atc cgc     288
   Ala Arg Phe Val Arg Leu Lys Gly Leu Thr Asn Lys Arg Asp Ile Arg
                   85                  90                  95
   gtc agc acc gcc aac ggg cgg atg gtt ctg acc gtc acc gat gat gat     336
   Val Ser Thr Ala Asn Gly Arg Met Val Leu Thr Val Thr Asp Asp Asp
               100                 105                 110
   ctg gtc cgc gta aat atg ggc gaa ccc aac ttc gaa cct tcc gcc gtg     384
   Leu Val Arg Val Asn Met Gly Glu Pro Asn Phe Glu Pro Ser Ala Val
           115                 120                 125
   ccg ttt cgc gct aac aaa gcg gaa aag acc tat att atg cgc gcc gcc     432
   Pro Phe Arg Ala Asn Lys Ala Glu Lys Thr Tyr Ile Met Arg Ala Ala
       130                 135                 140
   gag cag aca atc tta tgc ggc gtg gtg tcg atg gga aat ccg cat tgc     480
   Glu Gln Thr Ile Leu Cys Gly Val Val Ser Met Gly Asn Pro His Cys
   145                 150                 155                 160
   gtg att cag gtc gat gat gtc gat acc gcg gcg gta gaa acg ctt ggt     528
   Val Ile Gln Val Asp Asp Val Asp Thr Ala Ala Val Glu Thr Leu Gly
                   165                 170                 175
   cct gtt ctg gaa agc cac gag cgt ttt ccg gag cgc gcc aat atc ggt     576
   Pro Val Leu Glu Ser His Glu Arg Phe Pro Glu Arg Ala Asn Ile Gly
               180                 185                 190
   ttt atg caa gtg gtt aag cgc gag cat att cgt tta cgc gtt tat gag     624
   Phe Met Gln Val Val Lys Arg Glu His Ile Arg Leu Arg Val Tyr Glu
           195                 200                 205
   cgt ggg gca gga gaa acc cag gcc tgc ggc agc ggc gcg tgt gcg gcg     672
   Arg Gly Ala Gly Glu Thr Gln Ala Cys Gly Ser Gly Ala Cys Ala Ala
       210                 215                 220
   gtt gca gta ggg att cag caa ggt ttg ctg gcc gaa gaa gta cgc gtg     720
   Val Ala Val Gly Ile Gln Gln Gly Leu Leu Ala Glu Glu Val Arg Val
   225                 230                 235                 240
   gaa ctc ccc ggc ggt cgt ctt gat atc gcc tgg aaa ggt ccg ggt cac     768
   Glu Leu Pro Gly Gly Arg Leu Asp Ile Ala Trp Lys Gly Pro Gly His
                   245                 250                 255
   ccg tta tat atg act ggc ccg gcg gta cat gtc tac gac gga ttt att     816
   Pro Leu Tyr Met Thr Gly Pro Ala Val His Val Tyr Asp Gly Phe Ile
               260                 265                 270
   cat cta tga                                                         825
   His Leu
<210> 8
   <211> 274
   <212> PRT
   <213> Escherichia coli
<400> 8
   Met Gln Phe Ser Lys Met His Gly Leu Gly Asn Asp Phe Met Val Val
   1               5                   10                  15
   Asp Ala Val Thr Gln Asn Val Phe Phe Ser Pro Glu Leu Ile Arg Arg
               20                  25                  30
   Leu Ala Asp Arg His Leu Gly Val Gly Phe Asp Gln Leu Leu Val Val
           35                  40                  45
   Glu Pro Pro Tyr Asp Pro Glu Leu Asp Phe His Tyr Arg Ile Phe Asn
       50                  55                  60
   Ala Asp Gly Ser Glu Val Ala Gln Cys Gly Asn Gly Ala Arg Cys Phe
   65                  70                  75                  80
   Ala Arg Phe Val Arg Leu Lys Gly Leu Thr Asn Lys Arg Asp Ile Arg
                   85                  90                  95
   Val Ser Thr Ala Asn Gly Arg Met Val Leu Thr Val Thr Asp Asp Asp
               100                 105                 110
   Leu Val Arg Val Asn Met Gly Glu Pro Asn Phe Glu Pro Ser Ala Val
           115                 120                 125
   Pro Phe Arg Ala Asn Lys Ala Glu Lys Thr Tyr Ile Met Arg Ala Ala
       130                 135                 140
   Glu Gln Thr Ile Leu Cys Gly Val Val Ser Met Gly Asn Pro His Cys
   145                 150                 155                 160
   Val Ile Gln Val Asp Asp Val Asp Thr Ala Ala Val Glu Thr Leu Gly
                   165                 170                 175
   Pro Val Leu Glu Ser His Glu Arg Phe Pro Glu Arg Ala Asn Ile Gly
               180                 185                 190
   Phe Met Gln Val Val Lys Arg Glu His Ile Arg Leu Arg Val Tyr Glu
           195                 200                 205
   Arg Gly Ala Gly Glu Thr Gln Ala Cys Gly Ser Gly Ala Cys Ala Ala
       210                 215                 220
   Val Ala Val Gly Ile Gln Gln Gly Leu Leu Ala Glu Glu Val Arg Val
   225                 230                 235                 240
   Glu Leu Pro Gly Gly Arg Leu Asp Ile Ala Trp Lys Gly Pro Gly His
                   245                 250                 255
   Pro Leu Tyr Met Thr Gly Pro Ala Val His Val Tyr Asp Gly Phe Ile
               260                 265                 270
   His Leu
<210> 9
   <211> 963
   <212> DNA
   <213> Corynebacterium glutamicum
<220>
   <221> CDS
   <222> (1)..(963)
<400> 9
   atg acc aac atc cgc gta gct atc gtg ggc tac gga aac ctg gga cgc        48
   Met Thr Asn Ile Arg Val Ala Ile Val Gly Tyr Gly Asn Leu Gly Arg
   1               5                   10                  15
   agc gtc gaa aag ctt att gcc aag cag ccc gac atg gac ctt gta gga        96
   Ser Val Glu Lys Leu Ile Ala Lys Gln Pro Asp Met Asp Leu Val Gly
               20                  25                  30
   atc ttc tcg cgc cgg gcc acc ctc gac aca aag acg cca gtc ttt gat       144
   Ile Phe Ser Arg Arg Ala Thr Leu Asp Thr Lys Thr Pro Val Phe Asp
           35                  40                  45
   gtc gcc gac gtg gac aag cac gcc gac gac gtg gac gtg ctg ttc ctg       192
   Val Ala Asp Val Asp Lys His Ala Asp Asp Val Asp Val Leu Phe Leu
       50                  55                  60
   tgc atg ggc tcc gcc acc gac atc cct gag cag gca cca aag ttc gcg       240
   Cys Met Gly Ser Ala Thr Asp Ile Pro Glu Gln Ala Pro Lys Phe Ala
   65                  70                  75                  80
   cag ttc gcc tgc acc gta gac acc tac gac aac cac cgc gac atc cca        288
   Gln Phe Ala Cys Thr Val Asp Thr Tyr Asp Asn His Arg Asp Ile Pro
                   85                  90                  95
   cgc cac cgc cag gtc atg aac gaa gcc gcc acc gca gcc ggc aac gtt        336
   Arg His Arg Gln Val Met Asn Glu Ala Ala Thr Ala Ala Gly Asn Val
               100                 105                 110
   gca ctg gtc tct acc ggc tgg gat cca gga atg ttc tcc atc aac cgc        384
   Ala Leu Val Ser Thr Gly Trp Asp Pro Gly Met Phe Ser Ile Asn Arg
           115                 120                 125
   gtc tac gca gcg gca gtc tta gcc gag cac cag cag cac acc ttc tgg        432
   Val Tyr Ala Ala Ala Val Leu Ala Glu His Gln Gln His Thr Phe Trp
       130                 135                 140
   ggc cca ggt ttg tca cag ggc cac tcc gat gct ttg cga cgc atc cct        480
   Gly Pro Gly Leu Ser Gln Gly His Ser Asp Ala Leu Arg Arg Ile Pro
   145                 150                 155                 160
   ggc gtt caa aag gca gtc cag tac acc ctc cca tcc gaa gac gcc ctg        528
   Gly Val Gln Lys Ala Val Gln Tyr Thr Leu Pro Ser Glu Asp Ala Leu
                   165                 170                 175
   gaa aag gcc cgc cgc ggc gaa gcc ggc gac ctt acc gga aag caa acc        576
   Glu Lys Ala Arg Arg Gly Glu Ala Gly Asp Leu Thr Gly Lys Gln Thr
               180                 185                 190
   cac aag cgc caa tgc ttc gtg gtt gcc gac gcg gcc gat cac gag cgc        624
   His Lys Arg Gln Cys Phe Val Val Ala Asp Ala Ala Asp His Glu Arg
           195                 200                 205
   atc gaa aac gac atc cgc acc atg cct gat tac ttc gtt ggc tac gaa        672
   Ile Glu Asn Asp Ile Arg Thr Met Pro Asp Tyr Phe Val Gly Tyr Glu
       210                 215                 220
   gtc gaa gtc aac ttc atc gac gaa gca acc ttc gac tcc gag cac acc        720
   Val Glu Val Asn Phe Ile Asp Glu Ala Thr Phe Asp Ser Glu His Thr
   225                 230                 235                 240
   ggc atg cca cac ggt ggc cac gtg att acc acc ggc gac acc ggt ggc        768
   Gly Met Pro His Gly Gly His Val Ile Thr Thr Gly Asp Thr Gly Gly
                   245                 250                 255
   ttc aac cac acc gtg gaa tac atc ctc aag ctg gac cga aac cca gat        816
   Phe Asn His Thr Val Glu Tyr Ile Leu Lys Leu Asp Arg Asn Pro Asp
               260                 265                 270
   ttc acc gct tcc tca cag atc gct ttc ggt cgc gca gct cac cgc atg        864
   Phe Thr Ala Ser Ser Gln Ile Ala Phe Gly Arg Ala Ala His Arg Met
           275                 280                 285
   aag cag cag ggc caa agc gga gct ttc acc gtc ctc gaa gtt gct cca        912
   Lys Gln Gln Gly Gln Ser Gly Ala Phe Thr Val Leu Glu Val Ala Pro
       290                 295                 300
   tac ctg ctc tcc cca gag aac ttg gac gat ctg atc gca cgc gac gtc        960
   Tyr Leu Leu Ser Pro Glu Asn Leu Asp Asp Leu Ile Ala Arg Asp Val
   305                 310                 315                 320
   taa                                                                    963
<210> 10
   <211> 320
   <212> PRT
   <213> Corynebacterium glutamicum
<400> 10
   Met Thr Asn Ile Arg Val Ala Ile Val Gly Tyr Gly Asn Leu Gly Arg
   1               5                   10                  15
   Ser Val Glu Lys Leu Ile Ala Lys Gln Pro Asp Met Asp Leu Val Gly
               20                  25                  30
   Ile Phe Ser Arg Arg Ala Thr Leu Asp Thr Lys Thr Pro Val Phe Asp
           35                  40                  45
   Val Ala Asp Val Asp Lys His Ala Asp Asp Val Asp Val Leu Phe Leu
       50                  55                  60
   Cys Met Gly Ser Ala Thr Asp Ile Pro Glu Gln Ala Pro Lys Phe Ala
   65                  70                  75                  80
   Gln Phe Ala Cys Thr Val Asp Thr Tyr Asp Asn His Arg Asp Ile Pro
                   85                  90                  95
   Arg His Arg Gln Val Met Asn Glu Ala Ala Thr Ala Ala Gly Asn Val
               100                 105                 110
   Ala Leu Val Ser Thr Gly Trp Asp Pro Gly Met Phe Ser Ile Asn Arg
           115                 120                 125
   Val Tyr Ala Ala Ala Val Leu Ala Glu His Gln Gln His Thr Phe Trp
       130                 135                 140
   Gly Pro Gly Leu Ser Gln Gly His Ser Asp Ala Leu Arg Arg Ile Pro
   145                 150                 155                 160
   Gly Val Gln Lys Ala Val Gln Tyr Thr Leu Pro Ser Glu Asp Ala Leu
                   165                 170                 175
   Glu Lys Ala Arg Arg Gly Glu Ala Gly Asp Leu Thr Gly Lys Gln Thr
               180                 185                 190
   His Lys Arg Gln Cys Phe Val Val Ala Asp Ala Ala Asp His Glu Arg
           195                 200                 205
   Ile Glu Asn Asp Ile Arg Thr Met Pro Asp Tyr Phe Val Gly Tyr Glu
       210                 215                 220
   Val Glu Val Asn Phe Ile Asp Glu Ala Thr Phe Asp Ser Glu His Thr
   225                 230                 235                 240
   Gly Met Pro His Gly Gly His Val Ile Thr Thr Gly Asp Thr Gly Gly
                   245                 250                 255
   Phe Asn His Thr Val Glu Tyr Ile Leu Lys Leu Asp Arg Asn Pro Asp
               260                 265                 270
   Phe Thr Ala Ser Ser Gln Ile Ala Phe Gly Arg Ala Ala His Arg Met
           275                 280                 285
   Lys Gln Gln Gly Gln Ser Gly Ala Phe Thr Val Leu Glu Val Ala Pro
       290                 295                 300
   Tyr Leu Leu Ser Pro Glu Asn Leu Asp Asp Leu Ile Ala Arg Asp Val
   305                 310                 315                 320
<210> 11
   <211> 963
   <212> DNA
   <213> Brevibacterium lactofermentum
<220>
   <221> CDS
   <222> (1)..(963)
<400> 11
   atg acc aac atc cgc gta gct atc gta ggc tac gga aac ctg gga cgc        48
   Met Thr Asn Ile Arg Val Ala Ile Val Gly Tyr Gly Asn Leu Gly Arg
   1               5                   10                  15
   agc gtc gaa aag ctt att gcc aag cag ccc gac atg gac ctt gta gga        96
   Ser Val Glu Lys Leu Ile Ala Lys Gln Pro Asp Met Asp Leu Val Gly
               20                  25                  30
   atc ttc tcg cgc cgg gcc acc ctc gac aca aag acg cca gtc ttt gat       144
   Ile Phe Ser Arg Arg Ala Thr Leu Asp Thr Lys Thr Pro Val Phe Asp
           35                  40                  45
   gtc gcc gac gtg gac aag cac gcc gac gac gtg gac gtg ctg ttc ctg       192
   Val Ala Asp Val Asp Lys His Ala Asp Asp Val Asp Val Leu Phe Leu
       50                  55                  60
   tgc atg ggc tcc gcc acc gac atc cct gag cag gca cca aag ttc gcg       240
   Cys Met Gly Ser Ala Thr Asp Ile Pro Glu Gln Ala Pro Lys Phe Ala
   65                  70                  75                  80
   cag ttc gcc tgc acc gta gac acc tac gac aac cac cgc gac atc cca        288
   Gln Phe Ala Cys Thr Val Asp Thr Tyr Asp Asn His Arg Asp Ile Pro
                   85                  90                  95
   cgc cac cgc cag gtc atg aac gaa gcc gcc acc gca gcc ggc aac gtt        336
   Arg His Arg Gln Val Met Asn Glu Ala Ala Thr Ala Ala Gly Asn Val
               100                 105                 110
   gca ctg gtc tct acc ggc tgg gat cca gga atg ttc tcc atc aac cgc        384
   Ala Leu Val Ser Thr Gly Trp Asp Pro Gly Met Phe Ser Ile Asn Arg
           115                 120                 125
   gtc tac gca gcg gca gtc tta gcc gag cac cag cag cac acc ttc tgg        432
   Val Tyr Ala Ala Ala Val Leu Ala Glu His Gln Gln His Thr Phe Trp
       130                 135                 140
   ggc cca ggt ttg tca cag ggc cac tcc gat gct ttg cga cgc atc cct        480
   Gly Pro Gly Leu Ser Gln Gly His Ser Asp Ala Leu Arg Arg Ile Pro
   145                 150                 155                 160
   ggc gtt caa aag gcc gtc cag tac acc ctc cca tcc gaa gaa gcc ctg        528
   Gly Val Gln Lys Ala Val Gln Tyr Thr Leu Pro Ser Glu Glu Ala Leu
                   165                 170                 175
   gaa aag gcc cgc cgt ggc gaa gcc ggc gac ctc acc gga aag caa acc        576
   Glu Lys Ala Arg Arg Gly Glu Ala Gly Asp Leu Thr Gly Lys Gln Thr
               180                 185                 190
   cac aag cgc caa tgc ttc gtg gtt gcc gac gcg gcc gac cac gag cgc        624
   His Lys Arg Gln Cys Phe Val Val Ala Asp Ala Ala Asp His Glu Arg
           195                 200                 205
   atc gaa aac gac atc cgc acc atg cct gat tac ttc gtt ggc tac gaa        672
   Ile Glu Asn Asp Ile Arg Thr Met Pro Asp Tyr Phe Val Gly Tyr Glu
       210                 215                 220
   gtc gaa gtc aac ttc atc gac gaa gca acc ttc gac gcc gag cac acc        720
   Val Glu Val Asn Phe Ile Asp Glu Ala Thr Phe Asp Ala Glu His Thr
   225                 230                 235                 240
   ggc atg cca cac ggc gga cac gtg atc acc acc ggc gac acc ggt ggc        768
   Gly Met Pro His Gly Gly His Val Ile Thr Thr Gly Asp Thr Gly Gly
                   245                 250                 255
   ttc aac cac acc gtg gaa tac atc ctg aag ctg gac cga aac cca gat        816
   Phe Asn His Thr Val Glu Tyr Ile Leu Lys Leu Asp Arg Asn Pro Asp
               260                 265                 270
   ttc acc gct tct tca cag atc gct ttc ggc cgc gca gct cac cgc atg        864
   Phe Thr Ala Ser Ser Gln Ile Ala Phe Gly Arg Ala Ala His Arg Met
           275                 280                 285
   aag cag cag ggc caa agc ggt gcc ttc acc gtc ctc gaa gtt gct cca        912
   Lys Gln Gln Gly Gln Ser Gly Ala Phe Thr Val Leu Glu Val Ala Pro
       290                 295                 300
   tac ttg ctc tcc ccg gag aac ttg gat gat ctg atc gca cgc gac gtc        960
   Tyr Leu Leu Ser Pro Glu Asn Leu Asp Asp Leu Ile Ala Arg Asp Val
   305                 310                 315                 320
   taa                                                                    963
<210> 12
   <211> 320
   <212> PRT
   <213> Brevibacterium lactofermentum
<400> 12
   Met Thr Asn Ile Arg Val Ala Ile Val Gly Tyr Gly Asn Leu Gly Arg
   1               5                   10                  15
   Ser Val Glu Lys Leu Ile Ala Lys Gln Pro Asp Met Asp Leu Val Gly
               20                  25                  30
   Ile Phe Ser Arg Arg Ala Thr Leu Asp Thr Lys Thr Pro Val Phe Asp
           35                  40                  45
   Val Ala Asp Val Asp Lys His Ala Asp Asp Val Asp Val Leu Phe Leu
       50                  55                  60
   Cys Met Gly Ser Ala Thr Asp Ile Pro Glu Gln Ala Pro Lys Phe Ala
   65                  70                  75                  80
   Gln Phe Ala Cys Thr Val Asp Thr Tyr Asp Asn His Arg Asp Ile Pro
                   85                  90                  95
   Arg His Arg Gln Val Met Asn Glu Ala Ala Thr Ala Ala Gly Asn Val
               100                 105                 110
   Ala Leu Val Ser Thr Gly Trp Asp Pro Gly Met Phe Ser Ile Asn Arg
           115                 120                 125
   Val Tyr Ala Ala Ala Val Leu Ala Glu His Gln Gln His Thr Phe Trp
       130                 135                 140
   Gly Pro Gly Leu Ser Gln Gly His Ser Asp Ala Leu Arg Arg Ile Pro
   145                 150                 155                 160
   Gly Val Gln Lys Ala Val Gln Tyr Thr Leu Pro Ser Glu Glu Ala Leu
                   165                 170                 175
   Glu Lys Ala Arg Arg Gly Glu Ala Gly Asp Leu Thr Gly Lys Gln Thr
               180                 185                 190
   His Lys Arg Gln Cys Phe Val Val Ala Asp Ala Ala Asp His Glu Arg
           195                 200                 205
   Ile Glu Asn Asp Ile Arg Thr Met Pro Asp Tyr Phe Val Gly Tyr Glu
       210                 215                 220
   Val Glu Val Asn Phe Ile Asp Glu Ala Thr Phe Asp Ala Glu His Thr
   225                 230                 235                 240
   Gly Met Pro His Gly Gly His Val Ile Thr Thr Gly Asp Thr Gly Gly
                   245                 250                 255
   Phe Asn His Thr Val Glu Tyr Ile Leu Lys Leu Asp Arg Asn Pro Asp
               260                 265                 270
   Phe Thr Ala Ser Ser Gln Ile Ala Phe Gly Arg Ala Ala His Arg Met
           275                 280                 285
   Lys Gln Gln Gly Gln Ser Gly Ala Phe Thr Val Leu Glu Val Ala Pro
       290                 295                 300
   Tyr Leu Leu Ser Pro Glu Asn Leu Asp Asp Leu Ile Ala Arg Asp Val
   305                 310                 315                 320
<210> 13
   <211> 963
   <212> DNA
   <213> Corynebacterium efficiens
<220>
   <221> CDS
   <222> (1)..(963)
<400> 13
   atg tcg aag atc cgc gca gca atc gtt ggt tat gga aat ctg ggg aag       48
   Met Ser Lys Ile Arg Ala Ala Ile Val Gly Tyr Gly Asn Leu Gly Lys
   1               5                   10                  15
   agc gtc gag aag ctc atc gtc cag caa ccg gac atg gaa ctg gtg ggg       96
   Ser Val Glu Lys Leu Ile Val Gln Gln Pro Asp Met Glu Leu Val Gly
               20                  25                  30
   atc ttc tcc cgc cgc gac acc ctg gac acc gac acc ccc gtg ttc aac      144
   Ile Phe Ser Arg Arg Asp Thr Leu Asp Thr Asp Thr Pro Val Phe Asn
           35                  40                  45
   gtc gcc gag acg gag aag cac acc ggc gat gtt gat ctc ctc ttc ctc      192
   Val Ala Glu Thr Glu Lys His Thr Gly Asp Val Asp Leu Leu Phe Leu
       50                  55                  60
   tgc atg ggt tcc gcc act gac atc ccg gag cag gcc ccg ggt ttt gcg      240
   Cys Met Gly Ser Ala Thr Asp Ile Pro Glu Gln Ala Pro Gly Phe Ala
   65                  70                  75                  80
   gca ttc gcc tgc acc gtg gac acc tat gac aac cac cgg gac atc ccg      288
   Ala Phe Ala Cys Thr Val Asp Thr Tyr Asp Asn His Arg Asp Ile Pro
                   85                  90                  95
   cgt cac cgt cag gtg atg gat gag gcc gcc cgt gcc gcc ggc aat gtc      336
   Arg His Arg Gln Val Met Asp Glu Ala Ala Arg Ala Ala Gly Asn Val
               100                 105                 110
   tct gtt gtc gcc acc ggt tgg gat ccg ggg atg ttc tcc atc aac cgc      384
   Ser Val Val Ala Thr Gly Trp Asp Pro Gly Met Phe Ser Ile Asn Arg
           115                 120                 125
   gtg tac ggc gca gcc ctg ctc gcc gat cac cag cag cac acc ttc tgg      432
   Val Tyr Gly Ala Ala Leu Leu Ala Asp His Gln Gln His Thr Phe Trp
       130                 135                 140
   gga ccg ggt ctg tcc cag ggc cac tcc gat gcc ttg cga cgc atc gac      480
   Gly Pro Gly Leu Ser Gln Gly His Ser Asp Ala Leu Arg Arg Ile Asp
   145                 150                 155                 160
   ggc gtc gag aag gcc gtc cag tac acc ctg cct tcc gag gat gcc ctg      528
   Gly Val Glu Lys Ala Val Gln Tyr Thr Leu Pro Ser Glu Asp Ala Leu
                   165                 170                 175
   gag aag gca cgc cgc ggt gag gct gag ggg ctg acc ggt aaa cag acc      576
   Glu Lys Ala Arg Arg Gly Glu Ala Glu Gly Leu Thr Gly Lys Gln Thr
               180                 185                 190
   cac aag cgt cag tgt ttc gtg gtc gcc ccg gag tcc gag cac gag cgc      624
   His Lys Arg Gln Cys Phe Val Val Ala Pro Glu Ser Glu His Glu Arg
           195                 200                 205
   atc gag aat gag atc cgc acc atg gct gac tac ttc gtc ggc tat gag      672
   Ile Glu Asn Glu Ile Arg Thr Met Ala Asp Tyr Phe Val Gly Tyr Glu
       210                 215                 220
   gtg gag gtc aac ttc atc gat gag gct acc ttc gat tcc gag cac acc      720
   Val Glu Val Asn Phe Ile Asp Glu Ala Thr Phe Asp Ser Glu His Thr
   225                 230                 235                 240
   gga atg ccc cac ggc ggt cat gtg atc acc acc ggt gac acc ggc ggt      768
   Gly Met Pro His Gly Gly His Val Ile Thr Thr Gly Asp Thr Gly Gly
                   245                 250                 255
   ttc cac cac act gtg gag tac acc ctg aag ctg gat cgc aac cct gac      816
   Phe His His Thr Val Glu Tyr Thr Leu Lys Leu Asp Arg Asn Pro Asp
               260                 265                 270
   ttc acc gcc tcc tcc cag att gcg ttc gga cgt gct gcc tac cga ctg      864
   Phe Thr Ala Ser Ser Gln Ile Ala Phe Gly Arg Ala Ala Tyr Arg Leu
           275                 280                 285
   aag gaa gca ggt cag gct ggg gca ttc acc gtt ctg gag gtc gct ccc      912
   Lys Glu Ala Gly Gln Ala Gly Ala Phe Thr Val Leu Glu Val Ala Pro
       290                 295                 300
   tac ctc ctg tcc ccg aca cca ctc gat gac ctg atc gcc cgc gac gtc      960
   Tyr Leu Leu Ser Pro Thr Pro Leu Asp Asp Leu Ile Ala Arg Asp Val
   305                 310                 315                 320
   tag                                                                  963
<210> 14
   <211> 320
   <212> PRT
   <213> Corynebacterium efficiens
<400> 14
   Met Ser Lys Ile Arg Ala Ala Ile Val Gly Tyr Gly Asn Leu Gly Lys
   1               5                   10                  15
   Ser Val Glu Lys Leu Ile Val Gln Gln Pro Asp Met Glu Leu Val Gly
               20                  25                  30
   Ile Phe Ser Arg Arg Asp Thr Leu Asp Thr Asp Thr Pro Val Phe Asn
           35                  40                  45
   Val Ala Glu Thr Glu Lys His Thr Gly Asp Val Asp Leu Leu Phe Leu
       50                  55                  60
   Cys Met Gly Ser Ala Thr Asp Ile Pro Glu Gln Ala Pro Gly Phe Ala
   65                  70                  75                  80
   Ala Phe Ala Cys Thr Val Asp Thr Tyr Asp Asn His Arg Asp Ile Pro
                   85                  90                  95
   Arg His Arg Gln Val Met Asp Glu Ala Ala Arg Ala Ala Gly Asn Val
               100                 105                 110
   Ser Val Val Ala Thr Gly Trp Asp Pro Gly Met Phe Ser Ile Asn Arg
           115                 120                 125
   Val Tyr Gly Ala Ala Leu Leu Ala Asp His Gln Gln His Thr Phe Trp
       130                 135                 140
   Gly Pro Gly Leu Ser Gln Gly His Ser Asp Ala Leu Arg Arg Ile Asp
   145                 150                 155                 160
   Gly Val Glu Lys Ala Val Gln Tyr Thr Leu Pro Ser Glu Asp Ala Leu
                   165                 170                 175
   Glu Lys Ala Arg Arg Gly Glu Ala Glu Gly Leu Thr Gly Lys Gln Thr
               180                 185                 190
   His Lys Arg Gln Cys Phe Val Val Ala Pro Glu Ser Glu His Glu Arg
           195                 200                 205
   Ile Glu Asn Glu Ile Arg Thr Met Ala Asp Tyr Phe Val Gly Tyr Glu
       210                 215                 220
   Val Glu Val Asn Phe Ile Asp Glu Ala Thr Phe Asp Ser Glu His Thr
   225                 230                 235                 240
   Gly Met Pro His Gly Gly His Val Ile Thr Thr Gly Asp Thr Gly Gly
                   245                 250                 255
   Phe His His Thr Val Glu Tyr Thr Leu Lys Leu Asp Arg Asn Pro Asp
               260                 265                 270
   Phe Thr Ala Ser Ser Gln Ile Ala Phe Gly Arg Ala Ala Tyr Arg Leu
           275                 280                 285
   Lys Glu Ala Gly Gln Ala Gly Ala Phe Thr Val Leu Glu Val Ala Pro
       290                 295                 300
   Tyr Leu Leu Ser Pro Thr Pro Leu Asp Asp Leu Ile Ala Arg Asp Val
   305                 310                 315                 320
<210> 15
   <211> 987
   <212> DNA
   <213> Herminiimonas arsenicoxydans
<220>
   <221> CDS
   <222> (1)..(987)
<400> 15
   atg gat gaa aaa atc cgt att ggg gtt gcc gga tac ggt aac ctc ggt       48
   Met Asp Glu Lys Ile Arg Ile Gly Val Ala Gly Tyr Gly Asn Leu Gly
   1               5                   10                  15
   cgc ggg gtc gaa atg gcg atc gca cgc aac cct gat atg caa ctg gtc       96
   Arg Gly Val Glu Met Ala Ile Ala Arg Asn Pro Asp Met Gln Leu Val
               20                  25                  30
   ggc gtt ttc agc cgg cgc gat ccc gct agc atc gag ctt ttg acc cag      144
   Gly Val Phe Ser Arg Arg Asp Pro Ala Ser Ile Glu Leu Leu Thr Gln
           35                  40                  45
   gct gta ccc gtc cat aaa ttc gac gat atc gaa cag ttt cgc gac cag      192
   Ala Val Pro Val His Lys Phe Asp Asp Ile Glu Gln Phe Arg Asp Gln
       50                  55                  60
   att gac gtg ctc att ctg tgc ggc gga tca aag aac gat ctg ccc gaa      240
   Ile Asp Val Leu Ile Leu Cys Gly Gly Ser Lys Asn Asp Leu Pro Glu
   65                  70                  75                  80
   caa ggc cca gca ttg gct acc ttg ttc aac acg att gac agc ttt gat      288
   Gln Gly Pro Ala Leu Ala Thr Leu Phe Asn Thr Ile Asp Ser Phe Asp
                       85                  90                  95
   aca cac aat aaa att ccg gaa tat ttc gcc gcg atg gac agt gcc tca      336
   Thr His Asn Lys Ile Pro Glu Tyr Phe Ala Ala Met Asp Ser Ala Ser
                   100                 105                 110
   tgc gtt ggc gag cgc aca tcc atc atc tcg gta ggc tgg gat ccg ggc      384
   Cys Val Gly Glu Arg Thr Ser Ile Ile Ser Val Gly Trp Asp Pro Gly
               115                 120                 125
   gtg ttc tcg ctc aat cgt ctg ttc ggc gaa gcc att ttg cca gaa ggc      432
   Val Phe Ser Leu Asn Arg Leu Phe Gly Glu Ala Ile Leu Pro Glu Gly
       130                 135                 140
   gaa act tat acc ttc tgg ggt aaa ggt cta agt cag ggg cac tcg gac      480
   Glu Thr Tyr Thr Phe Trp Gly Lys Gly Leu Ser Gln Gly His Ser Asp
   145                 150                 155                 160
   gca ata cgc cgc gtg ccg ggc gtc aag gcc ggt gtg cag tac acg atc      528
   Ala Ile Arg Arg Val Pro Gly Val Lys Ala Gly Val Gln Tyr Thr Ile
                   165                 170                 175
   ccc tcg gct gag gcg atg gaa ctg gta cgc agt ggc agc cag ccc caa      576
   Pro Ser Ala Glu Ala Met Glu Leu Val Arg Ser Gly Ser Gln Pro Gln
               180                 185                 190
   ctg tcg aca cgc gag aaa cat act cgc gag tgt cat gtt gta ctc gaa      624
   Leu Ser Thr Arg Glu Lys His Thr Arg Glu Cys His Val Val Leu Glu
           195                 200                 205
   gcc ggt gcc gat gcc aaa gcg gtg gaa cat gcc atc gtc agc atg ccg      672
   Ala Gly Ala Asp Ala Lys Ala Val Glu His Ala Ile Val Ser Met Pro
       210                 215                 220
   gac tat ttc gcc gac tat gac acg acc gtg cac ttc atc agc gag gaa      720
   Asp Tyr Phe Ala Asp Tyr Asp Thr Thr Val His Phe Ile Ser Glu Glu
   225                 230                 235                 240
   gag tta cga agc aat cac tct gcc atg cca cac gga ggc ttc gtt atc      768
   Glu Leu Arg Ser Asn His Ser Ala Met Pro His Gly Gly Phe Val Ile
                   245                 250                 255
   cgc agc ggc cag aca gga gat ggc agc aaa cag gta ata gag tat tcg      816
   Arg Ser Gly Gln Thr Gly Asp Gly Ser Lys Gln Val Ile Glu Tyr Ser
               260                 265                 270
   ctc aag ctc ggt agc aat cct gaa ttt aca gcc gcg gta cta gtc gct      864
   Leu Lys Leu Gly Ser Asn Pro Glu Phe Thr Ala Ala Val Leu Val Ala
           275                 280                 285
   tac gcc cgc gct gct ttc cgc ctc cac aaa aaa gga gtg cac gga gcg      912
   Tyr Ala Arg Ala Ala Phe Arg Leu His Lys Lys Gly Val His Gly Ala
       290                 295                 300
   cac agc gtg ctg gat atc gct ccg ggc ctt ctg tca ccc aaa agt ccg      960
   His Ser Val Leu Asp Ile Ala Pro Gly Leu Leu Ser Pro Lys Ser Pro
   305                 310                 315                 320
   gct cag ttg cgc aaa gag ttg ctc tga                                  987
   Ala Gln Leu Arg Lys Glu Leu Leu
                   325
<210> 16
   <211> 328
   <212> PRT
   <213> Herminiimonas arsenicoxydans
<400> 16
   Met Asp Glu Lys Ile Arg Ile Gly Val Ala Gly Tyr Gly Asn Leu Gly
   1               5                   10                  15
   Arg Gly Val Glu Met Ala Ile Ala Arg Asn Pro Asp Met Gln Leu Val
               20                  25                  30
   Gly Val Phe Ser Arg Arg Asp Pro Ala Ser Ile Glu Leu Leu Thr Gln
           35                  40                  45
   Ala Val Pro Val His Lys Phe Asp Asp Ile Glu Gln Phe Arg Asp Gln
       50                  55                  60
   Ile Asp Val Leu Ile Leu Cys Gly Gly Ser Lys Asn Asp Leu Pro Glu
   65                  70                  75                  80
   Gln Gly Pro Ala Leu Ala Thr Leu Phe Asn Thr Ile Asp Ser Phe Asp
                   85                  90                  95
   Thr His Asn Lys Ile Pro Glu Tyr Phe Ala Ala Met Asp Ser Ala Ser
               100                 105                 110
   Cys Val Gly Glu Arg Thr Ser Ile Ile Ser Val Gly Trp Asp Pro Gly
           115                 120                 125
   Val Phe Ser Leu Asn Arg Leu Phe Gly Glu Ala Ile Leu Pro Glu Gly
       130                 135                 140
   Glu Thr Tyr Thr Phe Trp Gly Lys Gly Leu Ser Gln Gly His Ser Asp
   145                 150                 155                 160
   Ala Ile Arg Arg Val Pro Gly Val Lys Ala Gly Val Gln Tyr Thr Ile
                   165                 170                 175
   Pro Ser Ala Glu Ala Met Glu Leu Val Arg Ser Gly Ser Gln Pro Gln
               180                 185                 190
   Leu Ser Thr Arg Glu Lys His Thr Arg Glu Cys His Val Val Leu Glu
           195                 200                 205
   Ala Gly Ala Asp Ala Lys Ala Val Glu His Ala Ile Val Ser Met Pro
       210                 215                 220
   Asp Tyr Phe Ala Asp Tyr Asp Thr Thr Val His Phe Ile Ser Glu Glu
   225                 230                 235                 240
   Glu Leu Arg Ser Asn His Ser Ala Met Pro His Gly Gly Phe Val Ile
                   245                 250                 255
   Arg Ser Gly Gln Thr Gly Asp Gly Ser Lys Gln Val Ile Glu Tyr Ser
               260                 265                 270
   Leu Lys Leu Gly Ser Asn Pro Glu Phe Thr Ala Ala Val Leu Val Ala
           275                 280                 285
   Tyr Ala Arg Ala Ala Phe Arg Leu His Lys Lys Gly Val His Gly Ala
       290                 295                 300
   His Ser Val Leu Asp Ile Ala Pro Gly Leu Leu Ser Pro Lys Ser Pro
   305                 310                 315                 320
   Ala Gln Leu Arg Lys Glu Leu Leu
                   325
<210> 17
   <211> 900
   <212> DNA
   <213> Bacteroides thetaiotaomicron
<220>
   <221> CDS
   <222> (1)..(900)
<400> 17
   atg aaa aaa gta aga gca gct att gtc ggt tac ggc aat atc gga cac        48
   Met Lys Lys Val Arg Ala Ala Ile Val Gly Tyr Gly Asn Ile Gly His
   1               5                   10                  15
   tat gta ctt gaa gcg cta cag gca gcg cct gat ttc gaa ata gcc gga        96
   Tyr Val Leu Glu Ala Leu Gln Ala Ala Pro Asp Phe Glu Ile Ala Gly
               20                  25                  30
   gta gtt cgt cgt gca gga gca gag aac aag ccg gaa gag ttg gca aac       144
   Val Val Arg Arg Ala Gly Ala Glu Asn Lys Pro Glu Glu Leu Ala Asn
           35                  40                  45
   tat gca gta gtc aaa gat atc aaa gag ctg gaa gga gtg gaa gtg gcc       192
   Tyr Ala Val Val Lys Asp Ile Lys Glu Leu Glu Gly Val Glu Val Ala
       50                  55                  60
   atc ctc tgc aca ccg acc cgc agc gtt gag aaa tac gcg aaa gaa tat       240
   Ile Leu Cys Thr Pro Thr Arg Ser Val Glu Lys Tyr Ala Lys Glu Tyr
   65                  70                  75                  80
   ttg gca atg gga atc aac acg gtg gac agc ttc gac atc cac aca ggc       288
   Leu Ala Met Gly Ile Asn Thr Val Asp Ser Phe Asp Ile His Thr Gly
                   85                  90                  95
   atc gtt gac ctg cgc cgc acg ctg gat gcc acc gcc aaa gag cac aaa       336
   Ile Val Asp Leu Arg Arg Thr Leu Asp Ala Thr Ala Lys Glu His Lys
               100                 105                 110
   gcc gta tcc atc atc tcc gca gga tgg gat ccg gga agc gac tcg atc       384
   Ala Val Ser Ile Ile Ser Ala Gly Trp Asp Pro Gly Ser Asp Ser Ile
           115                 120                 125
   gta cgc acc atg ctc gaa gca atc gct ccg aaa ggc atc act tac acc       432
   Val Arg Thr Met Leu Glu Ala Ile Ala Pro Lys Gly Ile Thr Tyr Thr
       130                 135                 140
   aac ttc ggt ccc ggc atg agt atg gga cac acc gta gcc gta aaa gcg       480
   Asn Phe Gly Pro Gly Met Ser Met Gly His Thr Val Ala Val Lys Ala
   145                 150                 155                 160
   atc gac gga gtg aaa gcc gcc ctc tcc atg acg atc cct act gga acg       528
   Ile Asp Gly Val Lys Ala Ala Leu Ser Met Thr Ile Pro Thr Gly Thr
                   165                 170                 175
   gga atc cac cgc cgc atg gta tat atc gaa ctg aaa gac gga tac aag       576
   Gly Ile His Arg Arg Met Val Tyr Ile Glu Leu Lys Asp Gly Tyr Lys
               180                 185                 190
   ttt gaa gaa gta gcc gca gcc atc aag gca gac cct tac ttc gtg aac       624
   Phe Glu Glu Val Ala Ala Ala Ile Lys Ala Asp Pro Tyr Phe Val Asn
           195                 200                 205
   gac gag aca cat gta aaa ctt gtg ccc agc gta gac gca ctg ctc gat       672
   Asp Glu Thr His Val Lys Leu Val Pro Ser Val Asp Ala Leu Leu Asp
       210                 215                 220
   atg gga cac ggt gta aat ctg act cgc aag gga gtt tcc ggt aaa acg       720
   Met Gly His Gly Val Asn Leu Thr Arg Lys Gly Val Ser Gly Lys Thr
   225                 230                 235                 240
   cag aat cag ctg ttc gag ttc aat atg cgc atc aac aat ccg gca ctg       768
   Gln Asn Gln Leu Phe Glu Phe Asn Met Arg Ile Asn Asn Pro Ala Leu
                   245                 250                 255
   acc gca cag gta ctt gtg tgc gtg gca cgc gct tcc atg aag cag caa       816
   Thr Ala Gln Val Leu Val Cys Val Ala Arg Ala Ser Met Lys Gln Gln
               260                 265                 270
   ccg ggg tgc tac acc atg gta gaa gtc ccc gtc atc gac ctc ctt ccg       864
   Pro Gly Cys Tyr Thr Met Val Glu Val Pro Val Ile Asp Leu Leu Pro
           275                 280                 285
   ggc gac cgc gaa gag tgg atc gga cac ctg gta taa                       900
   Gly Asp Arg Glu Glu Trp Ile Gly His Leu Val
       290                 295
<210> 18
   <211> 299
   <212> PRT
   <213> Bacteroides thetaiotaomicron
<400> 18
   Met Lys Lys Val Arg Ala Ala Ile Val Gly Tyr Gly Asn Ile Gly His
   1               5                   10                  15
   Tyr Val Leu Glu Ala Leu Gln Ala Ala Pro Asp Phe Glu Ile Ala Gly
               20                  25                  30
   Val Val Arg Arg Ala Gly Ala Glu Asn Lys Pro Glu Glu Leu Ala Asn
           35                  40                  45
   Tyr Ala Val Val Lys Asp Ile Lys Glu Leu Glu Gly Val Glu Val Ala
       50                  55                  60
   Ile Leu Cys Thr Pro Thr Arg Ser Val Glu Lys Tyr Ala Lys Glu Tyr
   65                  70                  75                  80
   Leu Ala Met Gly Ile Asn Thr Val Asp Ser Phe Asp Ile His Thr Gly
                   85                  90                  95
   Ile Val Asp Leu Arg Arg Thr Leu Asp Ala Thr Ala Lys Glu His Lys
               100                 105                 110
   Ala Val Ser Ile Ile Ser Ala Gly Trp Asp Pro Gly Ser Asp Ser Ile
           115                 120                 125
   Val Arg Thr Met Leu Glu Ala Ile Ala Pro Lys Gly Ile Thr Tyr Thr
       130                 135                 140
   Asn Phe Gly Pro Gly Met Ser Met Gly His Thr Val Ala Val Lys Ala
   145                 150                 155                 160
   Ile Asp Gly Val Lys Ala Ala Leu Ser Met Thr Ile Pro Thr Gly Thr
                   165                 170                 175
   Gly Ile His Arg Arg Met Val Tyr Ile Glu Leu Lys Asp Gly Tyr Lys
               180                 185                 190
   Phe Glu Glu Val Ala Ala Ala Ile Lys Ala Asp Pro Tyr Phe Val Asn
           195                 200                 205
   Asp Glu Thr His Val Lys Leu Val Pro Ser Val Asp Ala Leu Leu Asp
       210                 215                 220
   Met Gly His Gly Val Asn Leu Thr Arg Lys Gly Val Ser Gly Lys Thr
   225                 230                 235                 240
   Gln Asn Gln Leu Phe Glu Phe Asn Met Arg Ile Asn Asn Pro Ala Leu
                   245                 250                 255
   Thr Ala Gln Val Leu Val Cys Val Ala Arg Ala Ser Met Lys Gln Gln
               260                 265                 270
   Pro Gly Cys Tyr Thr Met Val Glu Val Pro Val Ile Asp Leu Leu Pro
           275                 280                 285
   Gly Asp Arg Glu Glu Trp Ile Gly His Leu Val
       290                 295
<210> 19
   <211> 69
   <212> DNA
   <213> Artificial sequence
<220>
   <223> primer
<400> 19
   atgcagcagt tacagaacat tattgaaacc gcttttgaac gccgtgccga tctagacgct    60
   caagttagt                                                            69
<210> 20
   <211> 70
   <212> DNA
   <213> Artificial sequence
<220>
   <223> primer
<400> 20
   ttagtcgatg gtacgcagca gttcgttaat gccgactttg ccgcgagttt agatcttgaa      60
   gcctgctttt                                                             70
<210> 21
   <211> 1350
   <212> DNA
   <213> Escherichia coli
<220>
   <221> CDS
   <222> (1)..(1350)
<400> 21
   atg tct gaa att gtt gtc tcc aaa ttt ggc ggt acc agc gta gct gat      48
   Met Ser Glu Ile Val Val Ser Lys Phe Gly Gly Thr Ser Val Ala Asp
   1               5                   10                  15
   ttt gac gcc atg aac cgc agc gct gat att gtg ctt tct gat gcc aac      96
   Phe Asp Ala Met Asn Arg Ser Ala Asp Ile Val Leu Ser Asp Ala Asn
               20                  25                  30
   gtg cgt tta gtt gtc ctc tcg gct tct gct ggt atc act aat ctg ctg     144
   Val Arg Leu Val Val Leu Ser Ala Ser Ala Gly Ile Thr Asn Leu Leu
           35                  40                  45
   gtc gct tta gct gaa gga ctg gaa cct ggc gag cga ttc gaa aaa ctc     192
   Val Ala Leu Ala Glu Gly Leu Glu Pro Gly Glu Arg Phe Glu Lys Leu
       50                  55                  60
   gac gct atc cgc aac atc cag ttt gcc att ctg gaa cgt ctg cgt tac     240
   Asp Ala Ile Arg Asn Ile Gln Phe Ala Ile Leu Glu Arg Leu Arg Tyr
   65                  70                  75                  80
   ccg aac gtt atc cgt gaa gag att gaa cgt ctg ctg gag aac att act     288
   Pro Asn Val Ile Arg Glu Glu Ile Glu Arg Leu Leu Glu Asn Ile Thr
                   85                  90                  95
   gtt ctg gca gaa gcg gcg gcg ctg gca acg tct ccg gcg ctg aca gat     336
   Val Leu Ala Glu Ala Ala Ala Leu Ala Thr Ser Pro Ala Leu Thr Asp
               100                 105                 110
   gag ctg gtc agc cac ggc gag ctg atg tcg acc ctg ctg ttt gtt gag     384
   Glu Leu Val Ser His Gly Glu Leu Met Ser Thr Leu Leu Phe Val Glu
           115                 120                 125
   atc ctg cgc gaa cgc gat gtt cag gca cag tgg ttt gat gta cgt aaa     432
   Ile Leu Arg Glu Arg Asp Val Gln Ala Gln Trp Phe Asp Val Arg Lys
       130                 135                 140
   gtg atg cgt acc aac gac cga ttt ggt cgt gca gag cca gat ata gcc     480
   Val Met Arg Thr Asn Asp Arg Phe Gly Arg Ala Glu Pro Asp Ile Ala
   145                 150                 155                 160
   gcg ctg gcg gaa ctg gcc gcg ctg cag ctg ctc cca cgt ctc aat gaa     528
   Ala Leu Ala Glu Leu Ala Ala Leu Gln Leu Leu Pro Arg Leu Asn Glu
                   165                 170                 175
   ggc tta gtg atc acc cag gga ttt atc ggt agc gaa aat aaa ggt cgt     576
   Gly Leu Val Ile Thr Gln Gly Phe Ile Gly Ser Glu Asn Lys Gly Arg
               180                 185                 190
   aca acg acg ctt ggc cgt gga ggc agc gat tat acg gca gcc ttg ctg     624
   Thr Thr Thr Leu Gly Arg Gly Gly Ser Asp Tyr Thr Ala Ala Leu Leu
           195                 200                 205
   gcg gag gct tta cac gca tct cgt gtt gat atc tgg acc gac gtc ccg     672
   Ala Glu Ala Leu His Ala Ser Arg Val Asp Ile Trp Thr Asp Val Pro
       210                 215                 220
   ggc atc tac acc acc gat cca cgc gta gtt tcc gca gca aaa cgc att     720
   Gly Ile Tyr Thr Thr Asp Pro Arg Val Val Ser Ala Ala Lys Arg Ile
   225                 230                 235                 240
   gat gaa atc gcg ttt gcc gaa gcg gca gag atg gca act ttt ggt gca     768
   Asp Glu Ile Ala Phe Ala Glu Ala Ala Glu Met Ala Thr Phe Gly Ala
                   245                 250                 255
   aaa gta ctg cat ccg gca acg ttg cta ccc gca gta cgc agc gat atc     816
   Lys Val Leu His Pro Ala Thr Leu Leu Pro Ala Val Arg Ser Asp Ile
               260                 265                 270
   ccg gtc ttt gtc ggc tcc agc aaa gac cca cgc gca ggt ggt acg ctg     864
   Pro Val Phe Val Gly Ser Ser Lys Asp Pro Arg Ala Gly Gly Thr Leu
           275                 280                 285
   gtg tgc aat aaa act gaa aat ccg ccg ctg ttc cgc gct ctg gcg ctt     912
   Val Cys Asn Lys Thr Glu Asn Pro Pro Leu Phe Arg Ala Leu Ala Leu
       290                 295                 300
   cgt cgc aat cag act ctg ctc act ttg cac agc ctg aat atg ctg cat      960
   Arg Arg Asn Gln Thr Leu Leu Thr Leu His Ser Leu Asn Met Leu His
   305                 310                 315                 320
   tct cgc ggt ttc ctc gcg gaa gtt ttc ggc atc ctc gcg cgg cat aat     1008
   Ser Arg Gly Phe Leu Ala Glu Val Phe Gly Ile Leu Ala Arg His Asn
                   325                 330                 335
   att tcg gta gac tta atc acc acg tca gaa gtg agc gtg gca tta acc     1056
   Ile Ser Val Asp Leu Ile Thr Thr Ser Glu Val Ser Val Ala Leu Thr
               340                 345                 350
   ctt gat acc acc ggt tca acc tcc act ggc gat acg ttg ctg acg caa     1104
   Leu Asp Thr Thr Gly Ser Thr Ser Thr Gly Asp Thr Leu Leu Thr Gln
           355                 360                 365
   tct ctg ctg atg gag ctt tcc gca ctg tgt cgg gtg gag gtg gaa gaa     1152
   Ser Leu Leu Met Glu Leu Ser Ala Leu Cys Arg Val Glu Val Glu Glu
       370                 375                 380
   ggt ctg gcg ctg gtc gcg ttg att ggc aat gac ctg tca aaa gcc tgc     1200
   Gly Leu Ala Leu Val Ala Leu Ile Gly Asn Asp Leu Ser Lys Ala Cys
   385                 390                 395                 400
   ggc gtt ggc aaa gag gta ttc ggc gta ctg gaa ccg ttc aac att cgc     1248
   Gly Val Gly Lys Glu Val Phe Gly Val Leu Glu Pro Phe Asn Ile Arg
                   405                 410                 415
   atg att tgt tat ggc gca tcc agc cat aac ctg tgc ttc ctg gtg ccc     1296
   Met Ile Cys Tyr Gly Ala Ser Ser His Asn Leu Cys Phe Leu Val Pro
               420                 425                 430
   ggc gaa gat gcc gag cag gtg gtg caa aaa ctg cat agt aat ttg ttt     1344
   Gly Glu Asp Ala Glu Gln Val Val Gln Lys Leu His Ser Asn Leu Phe
           435                 440                 445
   gag taa                                                             1350
   Glu
<210> 22
   <211> 449
   <212> PRT
   <213> Escherichia coli
<400> 22
   Met Ser Glu Ile Val Val Ser Lys Phe Gly Gly Thr Ser Val Ala Asp
   1               5                   10                  15
   Phe Asp Ala Met Asn Arg Ser Ala Asp Ile Val Leu Ser Asp Ala Asn
               20                  25                  30
   Val Arg Leu Val Val Leu Ser Ala Ser Ala Gly Ile Thr Asn Leu Leu
           35                  40                  45
   Val Ala Leu Ala Glu Gly Leu Glu Pro Gly Glu Arg Phe Glu Lys Leu
       50                  55                  60
   Asp Ala Ile Arg Asn Ile Gln Phe Ala Ile Leu Glu Arg Leu Arg Tyr
   65                  70                  75                  80
   Pro Asn Val Ile Arg Glu Glu Ile Glu Arg Leu Leu Glu Asn Ile Thr
                   85                  90                  95
   Val Leu Ala Glu Ala Ala Ala Leu Ala Thr Ser Pro Ala Leu Thr Asp
               100                 105                 110
   Glu Leu Val Ser His Gly Glu Leu Met Ser Thr Leu Leu Phe Val Glu
           115                 120                 125
   Ile Leu Arg Glu Arg Asp Val Gln Ala Gln Trp Phe Asp Val Arg Lys
       130                 135                 140
   Val Met Arg Thr Asn Asp Arg Phe Gly Arg Ala Glu Pro Asp Ile Ala
   145                 150                 155                 160
   Ala Leu Ala Glu Leu Ala Ala Leu Gln Leu Leu Pro Arg Leu Asn Glu
                   165                 170                 175
   Gly Leu Val Ile Thr Gln Gly Phe Ile Gly Ser Glu Asn Lys Gly Arg
               180                 185                 190
   Thr Thr Thr Leu Gly Arg Gly Gly Ser Asp Tyr Thr Ala Ala Leu Leu
           195                 200                 205
   Ala Glu Ala Leu His Ala Ser Arg Val Asp Ile Trp Thr Asp Val Pro
       210                 215                 220
   Gly Ile Tyr Thr Thr Asp Pro Arg Val Val Ser Ala Ala Lys Arg Ile
   225                 230                 235                 240
   Asp Glu Ile Ala Phe Ala Glu Ala Ala Glu Met Ala Thr Phe Gly Ala
                   245                 250                 255
   Lys Val Leu His Pro Ala Thr Leu Leu Pro Ala Val Arg Ser Asp Ile
               260                 265                 270
   Pro Val Phe Val Gly Ser Ser Lys Asp Pro Arg Ala Gly Gly Thr Leu
           275                 280                 285
   Val Cys Asn Lys Thr Glu Asn Pro Pro Leu Phe Arg Ala Leu Ala Leu
       290                 295                 300
   Arg Arg Asn Gln Thr Leu Leu Thr Leu His Ser Leu Asn Met Leu His
   305                 310                 315                 320
   Ser Arg Gly Phe Leu Ala Glu Val Phe Gly Ile Leu Ala Arg His Asn
                   325                 330                 335
   Ile Ser Val Asp Leu Ile Thr Thr Ser Glu Val Ser Val Ala Leu Thr
               340                 345                 350
   Leu Asp Thr Thr Gly Ser Thr Ser Thr Gly Asp Thr Leu Leu Thr Gln
           355                 360                 365
   Ser Leu Leu Met Glu Leu Ser Ala Leu Cys Arg Val Glu Val Glu Glu
       370                 375                 380
   Gly Leu Ala Leu Val Ala Leu Ile Gly Asn Asp Leu Ser Lys Ala Cys
   385                 390                 395                 400
   Gly Val Gly Lys Glu Val Phe Gly Val Leu Glu Pro Phe Asn Ile Arg
                   405                 410                 415
   Met Ile Cys Tyr Gly Ala Ser Ser His Asn Leu Cys Phe Leu Val Pro
               420                 425                 430
   Gly Glu Asp Ala Glu Gln Val Val Gln Lys Leu His Ser Asn Leu Phe
           435                 440                 445
   Glu
<210> 23
   <211> 879
   <212> DNA
   <213> Escherichia coli
<220>
   <221> CDS
   <222> (1)..(879)
<400> 23
   atg ttc acg gga agt att gtc gcg att gtt act ccg atg gat gaa aaa       48
   Met Phe Thr Gly Ser Ile Val Ala Ile Val Thr Pro Met Asp Glu Lys
   1               5                   10                  15
   ggt aat gtc tgt cgg gct agc ttg aaa aaa ctg att gat tat cat gtc       96
   Gly Asn Val Cys Arg Ala Ser Leu Lys Lys Leu Ile Asp Tyr His Val
               20                  25                  30
   gcc agc ggt act tcg gcg atc gtt tct gtt ggc acc act ggc gag tcc      144
   Ala Ser Gly Thr Ser Ala Ile Val Ser Val Gly Thr Thr Gly Glu Ser
           35                  40                  45
   gct acc tta aat cat gac gaa cat gct gat gtg gtg atg atg acg ctg      192
   Ala Thr Leu Asn His Asp Glu His Ala Asp Val Val Met Met Thr Leu
       50                  55                  60
   gat ctg gct gat ggg cgc att ccg gta att gcc ggg acc ggc gct aac      240
   Asp Leu Ala Asp Gly Arg Ile Pro Val Ile Ala Gly Thr Gly Ala Asn
   65                  70                  75                  80
   gct act gcg gaa gcc att agc ctg acg cag cgc ttc aat gac agt ggt      288
   Ala Thr Ala Glu Ala Ile Ser Leu Thr Gln Arg Phe Asn Asp Ser Gly
                   85                  90                  95
   atc gtc ggc tgc ctg acg gta acc cct tac tac aat cgt ccg tcg caa      336
   Ile Val Gly Cys Leu Thr Val Thr Pro Tyr Tyr Asn Arg Pro Ser Gln
               100                 105                 110
   gaa ggt ttg tat cag cat ttc aaa gcc atc gct gag cat act gac ctg      384
   Glu Gly Leu Tyr Gln His Phe Lys Ala Ile Ala Glu His Thr Asp Leu
           115                 120                 125
   ccg caa att ctg tat aat gtg ccg tcc cgt act ggc tgc gat ctg ctc      432
   Pro Gln Ile Leu Tyr Asn Val Pro Ser Arg Thr Gly Cys Asp Leu Leu
       130                 135                 140
   ccg gaa acg gtg ggc cgt ctg gcg aaa gta aaa aat att atc gga atc      480
   Pro Glu Thr Val Gly Arg Leu Ala Lys Val Lys Asn Ile Ile Gly Ile
   145                 150                 155                 160
   aaa gag gca aca ggg aac tta acg cgt gta aac cag atc aaa gag ctg      528
   Lys Glu Ala Thr Gly Asn Leu Thr Arg Val Asn Gln Ile Lys Glu Leu
                   165                 170                 175
   gtt tca gat gat ttt gtt ctg ctg agc ggc gat gat gcg agc gcg ctg      576
   Val Ser Asp Asp Phe Val Leu Leu Ser Gly Asp Asp Ala Ser Ala Leu
               180                 185                 190
   gac ttc atg caa ttg ggc ggt cat ggg gtt att tcc gtt acg gct aac      624
   Asp Phe Met Gln Leu Gly Gly His Gly Val Ile Ser Val Thr Ala Asn
           195                 200                 205
   gtc gca gcg cgt gat atg gcc cag atg tgc aaa ctg gca gca gaa ggg      672
   Val Ala Ala Arg Asp Met Ala Gln Met Cys Lys Leu Ala Ala Glu Gly
       210                 215                 220
   cat ttt gcc gag gca cgc gtt att aat cag cgt ctg atg cca tta cac      720
   His Phe Ala Glu Ala Arg Val Ile Asn Gln Arg Leu Met Pro Leu His
   225                 230                 235                 240
   aac aaa cta ttt gtc gaa ccc aat cca atc ccg gtg aaa tgg gca tgt      768
   Asn Lys Leu Phe Val Glu Pro Asn Pro Ile Pro Val Lys Trp Ala Cys
                   245                 250                 255
   aag gaa ctg ggt ctt gtg gcg acc gat acg ctg cgc ctg cca atg aca      816
   Lys Glu Leu Gly Leu Val Ala Thr Asp Thr Leu Arg Leu Pro Met Thr
               260                 265                 270
   cca atc acc gac agt ggt cgt gag acg gtc aga gcg gcg ctt aag cat      864
   Pro Ile Thr Asp Ser Gly Arg Glu Thr Val Arg Ala Ala Leu Lys His
           275                 280                 285
   gcc ggt ttg ctg taa                                                  879
   Ala Gly Leu Leu
       290
<210> 24
   <211> 292
   <212> PRT
   <213> Escherichia coli
<400> 24
   Met Phe Thr Gly Ser Ile Val Ala Ile Val Thr Pro Met Asp Glu Lys
   1               5                   10                  15
   Gly Asn Val Cys Arg Ala Ser Leu Lys Lys Leu Ile Asp Tyr His Val
               20                  25                  30
   Ala Ser Gly Thr Ser Ala Ile Val Ser Val Gly Thr Thr Gly Glu Ser
           35                  40                  45
   Ala Thr Leu Asn His Asp Glu His Ala Asp Val Val Met Met Thr Leu
       50                  55                  60
   Asp Leu Ala Asp Gly Arg Ile Pro Val Ile Ala Gly Thr Gly Ala Asn
   65                  70                  75                  80
   Ala Thr Ala Glu Ala Ile Ser Leu Thr Gln Arg Phe Asn Asp Ser Gly
                   85                  90                  95
   Ile Val Gly Cys Leu Thr Val Thr Pro Tyr Tyr Asn Arg Pro Ser Gln
               100                 105                 110
   Glu Gly Leu Tyr Gln His Phe Lys Ala Ile Ala Glu His Thr Asp Leu
           115                 120                 125
   Pro Gln Ile Leu Tyr Asn Val Pro Ser Arg Thr Gly Cys Asp Leu Leu
       130                 135                 140
   Pro Glu Thr Val Gly Arg Leu Ala Lys Val Lys Asn Ile Ile Gly Ile
   145                 150                 155                 160
   Lys Glu Ala Thr Gly Asn Leu Thr Arg Val Asn Gln Ile Lys Glu Leu
                   165                 170                 175
   Val Ser Asp Asp Phe Val Leu Leu Ser Gly Asp Asp Ala Ser Ala Leu
               180                 185                 190
   Asp Phe Met Gln Leu Gly Gly His Gly Val Ile Ser Val Thr Ala Asn
           195                 200                 205
   Val Ala Ala Arg Asp Met Ala Gln Met Cys Lys Leu Ala Ala Glu Gly
       210                 215                 220
   His Phe Ala Glu Ala Arg Val Ile Asn Gln Arg Leu Met Pro Leu His
   225                 230                 235                 240
   Asn Lys Leu Phe Val Glu Pro Asn Pro Ile Pro Val Lys Trp Ala Cys
                   245                 250                 255
   Lys Glu Leu Gly Leu Val Ala Thr Asp Thr Leu Arg Leu Pro Met Thr
               260                 265                 270
   Pro Ile Thr Asp Ser Gly Arg Glu Thr Val Arg Ala Ala Leu Lys His
           275                 280                 285
   Ala Gly Leu Leu
       290
<210> 25
   <211> 822
   <212> DNA
   <213> Escherichia coli
<220>
   <221> CDS
   <222> (1)..(822)
<400> 25
   atg cat gat gca aac atc cgc gtt gcc atc gcg gga gcc ggg ggg cgt        48
   Met His Asp Ala Asn Ile Arg Val Ala Ile Ala Gly Ala Gly Gly Arg
   1               5                   10                  15
   atg ggc cgc cag ttg att cag gcg gcg ctg gca tta gag ggc gtg cag        96
   Met Gly Arg Gln Leu Ile Gln Ala Ala Leu Ala Leu Glu Gly Val Gln
               20                  25                  30
   ttg ggc gct gcg ctg gag cgt gaa gga tct tct tta ctg ggc agc gac        144
   Leu Gly Ala Ala Leu Glu Arg Glu Gly Ser Ser Leu Leu Gly Ser Asp
           35                  40                  45
   gcc ggt gag ctg gcc gga gcc ggg aaa aca ggc gtt acc gtg caa agc        192
   Ala Gly Glu Leu Ala Gly Ala Gly Lys Thr Gly Val Thr Val Gln Ser
       50                  55                  60
   agc ctc gat gcg gta aaa gat gat ttt gat gtg ttt atc gat ttt acc        240
   Ser Leu Asp Ala Val Lys Asp Asp Phe Asp Val Phe Ile Asp Phe Thr
   65                  70                  75                  80
   cgt ccg gaa ggt acg ctg aac cat ctc gct ttt tgt cgc cag cat ggc        288
   Arg Pro Glu Gly Thr Leu Asn His Leu Ala Phe Cys Arg Gln His Gly
                   85                  90                  95
   aaa ggg atg gtg atc ggc act acg ggg ttt gac gaa gcc ggt aaa caa        336
   Lys Gly Met Val Ile Gly Thr Thr Gly Phe Asp Glu Ala Gly Lys Gln
               100                 105                 110
   gca att cgt gac gcc gct gcc gat att gcg att gtc ttt gct gcc aat        384
   Ala Ile Arg Asp Ala Ala Ala Asp Ile Ala Ile Val Phe Ala Ala Asn
           115                 120                 125
   ttt agc gtt ggc gtt aac gtc atg ctt aag ctg ctg gag aaa gca gcc        432
   Phe Ser Val Gly Val Asn Val Met Leu Lys Leu Leu Glu Lys Ala Ala
       130                 135                 140
   aaa gtg atg ggt gac tac acc gat atc gaa att att gaa gca cat cat        480
   Lys Val Met Gly Asp Tyr Thr Asp Ile Glu Ile Ile Glu Ala His His
   145                 150                 155                 160
   aga cat aaa gtt gat gcg ccg tca ggc acc gca ctg gca atg gga gag        528
   Arg His Lys Val Asp Ala Pro Ser Gly Thr Ala Leu Ala Met Gly Glu
                   165                 170                 175
   gcg atc gcc cac gcc ctt gat aaa gat ctg aaa gat tgc gcg gtc tac        576
   Ala Ile Ala His Ala Leu Asp Lys Asp Leu Lys Asp Cys Ala Val Tyr
               180                 185                 190
   agt cgt gaa ggc cac acc ggt gaa cgt gtg cct ggc acc att ggt ttt        624
   Ser Arg Glu Gly His Thr Gly Glu Arg Val Pro Gly Thr Ile Gly Phe
           195                 200                 205
   gcc acc gtg cgt gca ggt gac atc gtt ggt gaa cat acc gcg atg ttt        672
   Ala Thr Val Arg Ala Gly Asp Ile Val Gly Glu His Thr Ala Met Phe
       210                 215                 220
   gcc gat att ggc gag cgt ctg gag atc acc cat aag gcg tcc agc cgt        720
   Ala Asp Ile Gly Glu Arg Leu Glu Ile Thr His Lys Ala Ser Ser Arg
   225                 230                 235                 240
   atg aca ttt gct aac ggc gcg gta aga tcg gct ttg tgg ttg agt ggt        768
   Met Thr Phe Ala Asn Gly Ala Val Arg Ser Ala Leu Trp Leu Ser Gly
                   245                 250                 255
   aag gaa agc ggt ctt ttt gat atg cga gat gta ctt gat ctc aat aat        816
   Lys Glu Ser Gly Leu Phe Asp Met Arg Asp Val Leu Asp Leu Asn Asn
               260                 265                 270
   ttg taa                                                                822
   Leu
<210> 26
   <211> 273
   <212> PRT
   <213> Escherichia coli
<400> 26
   Met His Asp Ala Asn Ile Arg Val Ala Ile Ala Gly Ala Gly Gly Arg
   1 5 10 15
   Met Gly Arg Gln Leu Ile Gln Ala Ala Leu Ala Leu Glu Gly Val Gln
   20 25 30
   Leu Gly Ala Ala Leu Glu Arg Glu Gly Ser Ser Leu Leu Gly Ser Asp
   35 40 45
   Ala Gly Glu Leu Ala Gly Ala Gly Lys Thr Gly Val Thr Val Gln Ser
   50 55 60
   Ser Leu Asp Ala Val Lys Asp Asp Phe Asp Val Phe Ile Asp Phe Thr
   65 70 75 80
   Arg Pro Glu Gly Thr Leu Asn His Leu Ala Phe Cys Arg Gln His Gly
   85 90 95
   Lys Gly Met Val Ile Gly Thr Thr Gly Phe Asp Glu Ala Gly Lys Gln
   100 105 110
   Ala Ile Arg Asp Ala Ala Ala Asp Ile Ala Ile Val Phe Ala Ala Asn
   115 120 125
   Phe Ser Val Gly Val Asn Val Met Leu Lys Leu Leu Glu Lys Ala Ala
   130 135 140
   Lys Val Met Gly Asp Tyr Thr Asp Ile Glu Ile Ile Glu Ala His His
   145 150 155 160
   Arg His Lys Val Asp Ala Pro Ser Gly Thr Ala Leu Ala Met Gly Glu
   165 170 175
   Ala Ile Ala His Ala Leu Asp Lys Asp Leu Lys Asp Cys Ala Val Tyr
   180 185 190
   Ser Arg Glu Gly His Thr Gly Glu Arg Val Pro Gly Thr Ile Gly Phe
   195 200 205
   Ala Thr Val Arg Ala Gly Asp Ile Val Gly Glu His Thr Ala Met Phe
   210 215 220
   Ala Asp Ile Gly Glu Arg Leu Glu Ile Thr His Lys Ala Ser Ser Arg
   225 230 235 240
   Met Thr Phe Ala Asn Gly Ala Val Arg Ser Ala Leu Trp Leu Ser Gly
   245 250 255
   Lys Glu Ser Gly Leu Phe Asp Met Arg Asp Val Leu Asp Leu Asn Asn
   260 265 270
   Leu